# EUROPEAN PATENT APPLICATION

(11) **EP 1 161 957 A1**
(43) Date of publication of application: **12.12.2001**
(21) Application number: 01111145.7
(22) Date of filing: 09.05.2001
(51) Int. Cl.: A61K 47/48, C12N 15/87, C07K 7/08, C07K 14/00

(54) **Complex for transferring an anionic substance of interest into a cell**

(30) Priority: 26.05.2000 EP 00440162; 27.02.2001 EP 01440049; 07.11.2000 US 246083 P; 23.03.2001 US 277982 P
(71) Applicant: TRANSGENE S.A., 67082 Strasbourg Cédex (FR)
(72) Inventor: Rittner, Karola, 67000 Strasbourg (FR); Jacobs, Eric, 67140 Stotzheim (FR)
(74) Representative: VOSSIUS & PARTNER

(57) **Abstract**

A peptide and a related complex for transferring an anionic substance of interest into a cell are disclosed wherein said peptide is a cationic peptide capable of binding to an anionic substance, capable to cause membrane disruption and which does not comprise acidic amino acid , preferably glutamic amino acid.

## Description

The present invention concerns a cationic peptide capable of causing membrane disruption, and complexes comprising said peptide and an anionic substance of interest, such as for example a nucleic acid molecule. These complexes are useful for delivering said anionic substance into a cell, particularly in gene therapy applications.

Gene therapy can be defined as the transfer of genetic material into a cell or an organism. The possibility of treating human disorders by gene therapy has changed in a few years from the stage of theoretical considerations to that of clinical applications. The first protocol applied to man was initiated in the USA in September 1990 on a patient suffering from adenine deaminase (ADA) deficiency. This first encouraging experiment has been followed by numerous new applications and promising clinical trials based on gene therapy which are currently ongoing (see for example clinical trials listed at http://cnetdb.nci.nih.gov/trialsrch.shtml or http://www.wiley.co.uk/genetherapy/clinical/).

Successful gene therapy depends principally on the efficient delivery of a therapeutic gene of interest to make its expression possible in cells of a living organism. Therapeutic genes can be transferred into cells using a wide variety of vectors resulting in either transient expression or permanent transformation of the host genome. During the past decade, a large number of viral, as well as non-viral, vectors has been developed for gene transfer (see for example Robbins et al., 1998, Tibtech 16, 35-40 and Rolland, 1998, Therapeutic Drug Carrier Systems 15, 143-198 for reviews).

Most of the intracellular gene delivery mechanisms used to date are viral vectors, especially adeno-, pox- and retroviral vectors (see Robbins et al., 1998, Tibtech, 16, 35-40 for a review). Nevertheless, said use of viruses suffers from a number of disadvantages: retroviral vectors cannot accommodate large-sized nucleotide sequences (e.g. the dystrophin gene which is around 13 kb), the retroviral genome is integrated into host cell DNA and may thus cause genetic changes in the recipient cell and infectious viral particles can disseminate within the organism or into the environment; adenoviral vectors can induce a strong immune response in treated patients (Mc Coy et al, 1995, Human Gene Therapy, 6, 1553-1560; Yang et al., 1996, Immunity, 1, 433-442).

Accordingly, in order to offer safer approach for intracellular nucleic acid delivery, non-viral systems have been proposed. Thus, in 1990, Wolff et al. (1990, Science, 247, 1465-1468) have shown that injection of naked RNA or DNA, without any special delivery system, directly into mouse skeletal muscle, results in expression of a reporter gene within the muscle cells. Nevertheless, although these results indicate that nucleic acid by itself is capable of crossing the plasma membrane of certain cells *in vivo,* the efficiency of the transfection, and thereby of the gene expression, observed in most of the cell types remains very limited due, in particular, to the polyanionic nature of nucleic acids which limits their passage through negatively-charged cell membranes.

In the same time, other groups (see Rolland, 1998, Therapeutic Drug Carrier Systems, 15, 143-198 for a review) proposed alternative synthetic systems using cationic lipids or cationic polymers in order to facilitate the introduction of anionic molecules such as nucleic acids into cells. These cationic compounds are capable of forming complexes with anionic molecules, thus tending to neutralize their negative charges and allowing to compact them in complexed form which favors their introduction into the cell. These non-viral delivery systems are, for example, based on receptor-mediated mechanisms (Perales et al., 1994, Eur. J. Biochem. 226, 255-266; Wagner et al., 1994, Advanced Drug Delivery Reviews, 14, 113-135), on polymer-mediated transfection such as polyamidoamine (Haensler et Szoka, 1993, Bioconjugate Chem., 4, 372-379), dendritic polymer (WO 95/24221), polyethylene imine or polypropylene imine (WO 96/02655), polylysine (US-A- 5 595 897 or FR 2 719 316) or on lipid-mediated transfection (Felgner et al., 1989, Nature, 337, 387-388) such as DOTMA (Felgner et al., 1987, PNAS, 84, 7413-7417), DOGS or Transfectam™ (Behr et al., 1989, PNAS, 86, 6982-6986), DMRIE or DORIE (Felgner et al., 1993, Methods 5, 67-75), DC-CHOL (Gao et Huang, 1991, BBRC, 179, 280-285), DOTAP™ (McLachlan et al., 1995, Gene Therapy, 2,674-622), Lipofectamine™ or glycerolipid compounds (see for example EP 901 463 and WO98/37916).

These non-viral systems present special advantages with respect to large-scale production, safety, low immunogenicity, and capacity to deliver large fragments of DNA.

Besides, analyses have shown that a major pathway for intracellular delivery of these non-viral systems is internalization into vesicles by endocytosis. Endocytosis is the natural process by which eukaryotic cells ingest segments of the plasma membrane in the form of small endocytosis vesicles, i.e. endosomes, entrapping extracellular fluid and molecular material, e.g. nucleic acid molecules. In cells, these endosomes fuse with lysosomes which are specialized sites of intracellular degradation. The lysosomes are acidic and contain a wide variety of degradative enzymes to digest the molecular contents of the endosomal vesicles. After endocytosis the internalized material is thus still separated from the cytoplasm by a membrane and therefore is not available for performing its desired function. Actually, said desired function, i.e. the desired therapeutic effect, depends in most of the nucleic acids transfer approaches on their delivery at least into the cytoplasm (e.g. for RNA) or rather into the nucleus of the cell (e.g. for DNA encoding a polypeptide or antisense oligonucleotides)where their functional effect can occur. Consequently, the internalized nucleic acid accumulation into endosomal vesicles strongly reduces the efficiency of nucleic acid functional transfer to the cell, and therefore the efficiency of gene therapy (Zabner et al.,1995, J. Biol. Chem., 270, 18997-19007).

Accordingly, the efficient delivery to and expression of genetic information within the cells of a living organism depend both on the capability of the delivery system to transfer the nucleic acid molecule into the cell and on its capability to promote nucleic acid escape from endosomal retention and degradation.

Once the delivery system has been taken up by cells via endocytosis, it must escape from the endosomal compartment for being localized in the cytoplasm or to migrate to the nucleus. The general strategy is to promote endosomolysis, e.g. by using fusogenic or membranolytic/endosomolytic peptides (see Mahato et al., 1999,Current Opinion in Mol. Therapeutics, 1, 226-243).

Some microorganisms (e.g. viruses) are naturally internalized via receptor-mediated endocytosis and have developed systems for escaping from the above-mentioned endosomal degradation. Based on this natural aptitude, gene transfer systems have been proposed including the endosome-destabilizing activity of replication-defective adenovirus particles or rhinovirus particles which were either added to the transfection medium (Cotten et al., 1992, Proc. Natl. Acad. Sci. USA, 89, 6094-6098) or directly linked to the delivery complex (Wu et al., 1994, J. Biol. Chem., 269, 11542-11546 ; US 5,928,944). Expression levels resulting from *in vivo* gene transfer with these systems, while promising, are still relatively low and further optimization is required. Additionally, synthetic systems have been generated. The best characterized synthetic peptides with fusogenic activity are derived from the first 23 amino acids of the N-terminal peptide of the HA2 subunit of influenza hemaglutinin (e.g. the INF peptide). At pH 7, this peptide preferentially assumes a random coil structure. At pH 5, an amphipathic alpha-helical conformation is favoured and the peptide becomes endosomolytic. Similarly, the synthetic peptide JTS-1 developped by Gottschalk et al. (1996, Gene Therapy, 3, 448-457) starts with the INF sequence GLFEA followed by an optimized peptide sequence. This JTS-1 peptide was shown to be capable of lysing calcein containing phosphatidylcholine liposomes at pH 5, more efficiently than at a pH 7.

However, the intracellular delivery of nucleic acids requires that said peptides combine their fusogenic activity with a nucleic acid complexing activity to form delivery complexes capable of transferring said nucleic acid into cells.

Some systems developed so far combine two distinct elements presenting said features (for example, WO 96/40958, WO 98/50078 or Gottschalk et al., 1996, Gene Therapy, 3, 448-457, Haensler & Szoka, 1993, Bioconjugate Chem., 4, 372-379). These two-components systems actually include peptides which have specificity for endosomal pH due to acidic residues (glutamic and aspartic amino acids). At neutral pH, the negatively charged carboxylic groups destabilize the structure of these peptides ; acidification of the carboxylic groups promotes multimerization of the peptides and /or membrane interaction leading to membrane destabilization and leakage. Wagner et al. (1999, Advanced Drug Delivery Reviews, 38, 279-289) have analyzed this pH specificity and have indicated that introduction of additional glutamic acids into peptides can enhance their pH specificity, and therefore their endosome disrupting property. However, said combined systems which retain the endosome disruptive properties of viral particles and are capable of associating with the nucleic acid molecule to form a complex must display a delicate balance between each distinct moieties (i.e. the nucleic acid-binding ligand and the synthetic membrane-destabilizing peptide) in order to promote intracellular nucleic acid transfer and to function under *in vitro* as well as *in vivo* conditions.

With the aim to propose a simplified system, Wyman et al. (1997, Biochemistry, 36, 3008-3017) developed a single-component system using a designed synthetic peptide, KALA, which can promote *in vitro* transfection of nucleic acid molecules and can cause membrane disruption. While positively charged hydrophilic lysine amino acid residues have been chosen to bind the nucleic acid molecule, glutamic amino acid residues are still maintained to provide the KALA peptide with pH specificity and thereby to guarantee its endosome disrupting property. Additionally, Gottschalk et al. (1996, Gene Therapy, 3, 448-457) have found that the disrupting activity of peptides is not the unique factor that determines gene transfer activity and that single amino acid substitutions in said peptide sequence can significantly decrease their disruptive property on the endosomal membrane, suggesting that these peptide systems require careful optimization at the risk of loosing at least one of the two required properties.

The available nucleic acid delivery systems are not yet satisfactory in terms of safety or efficiency for their utilization in *in vivo* gene therapy and require further optimization.

The technical problem underlying the present invention is the provision of improved methods and means for the delivery into cells of anionic substances, preferably of nucleic acid molecules, which are useful for therapy, preferably for gene therapy. This problem has been solved by the provision of the embodiments as characterized in the claims.

Accordingly, the present invention relates to a cationic peptide which is capable of causing membrane disruption and which does not comprise acidic amino acid, and more particularly said peptide does not comprise glutamic amino acid (Glu or E).

The peptide which has been identified in connection with the present invention is capable to cause cell membrane disruption, to bind an anionic substance, in particular a nucleic acid molecule, to enhance transfer of said anionic substance into a cell and eventually, to enhance expression of a genetic information into the transformed cell.

The term "peptide capable of causing membrane disruption" as used herein refers to a peptide which is capable of interacting with a membrane, particularly with a cellular membrane, and more particularly with an endosomal and/or lysosomal membrane, in such a manner that said interaction results in destabilizing and/or leaking of the membrane, and particularly in freeing the contents of the endosomes. Preferably, said interaction results in freeing the endosome and/or lysosome contents into the cytoplasm of the cell. The membrane disrupting property of the peptide can be easily measured for example by the method described in the appended Examples or in Olson et al.( 1979, Biochim. Biophys. Acta, 557, 19-23). The term "membrane" as used herein is intended to have the same meaning as commonly understood by one of ordinary skill in the art. Generally, it designates a mono or bi layer consisting mainly of lipids, and eventually contains proteins. Included are natural (e.g. membrane of the cells) and synthetic (e.g. liposomal) membranes. Preferred membranes are natural membranes such as for example cellular membranes, endosomal or lysosomal membranes, trans-Golgi network membranes, virus membranes, nuclear membranes.

The term "peptide", "amino acid residues" and "acidic amino acid residues" as used herein are intended to have the same meaning as commonly understood by one of ordinary skill in the art. Preferably, "peptide" refers to a polymer of amino acids residues that is less than 50 residues in length, more preferably less than 30 residues in length and most preferably less than 20 residues in length. In a preferred embodiment, the peptide of the present invention has a molecular weight of less than 5 kD and most preferably of less than 3 kD. Peptides according to the invention may be produced *de novo* by synthetic methods or by expression of the appropriate DNA fragment by recombinant DNA techniques in eukaryotic or prokaryotic cells. In a special embodiment, said peptide contains one or more non-hydrolyzable chemical moieties in place of those which exist in naturally occurring peptides, such as carboxyl moieties. In that special case, the naturally hydrolyzable moities are replaced by non-hydrolizable ones such as for example methylene moities. The present invention also encompasses analogs of the above described peptide, wherein at least one amino acid is replaced by another amino acid having similar properties, including retro or inverso peptides (WO95/24916). Additionally, the ligand moiety in use in the invention may include modifications of its original structure by way of substitution or addition of chemical moieties (e.g. glycosylation, alkylation, acetylation, amidation, phosphorylation, addition of sulfhydryl groups and the like). The present invention also contemplates modifications that render the peptides of the invention detectable. For this purpose, the peptides of the invention can be modified with a detectable moiety (i.e. a scintigraphic, radioactive, a fluorescent moiety, an enzyme, a dye label and the like). Suitable radioactive labels include but are not limited to Tc^{99m}, I¹²³ and In¹¹¹. Such labels can be attached to the peptide of the invention in a known manner, for example via a cysteine residue. Other techniques are described elsewhere. The labeled peptides of the invention may be used for diagnostic purposes (e.g. imaging of tumoral cells, of transformed cells, and the like).

In a special embodiment, the peptide of the invention is modified by addition of at least one cysteine residue at its N- and/or C-terminal extremities. This modification allows for example the formation of di-, tri- or multimeric association of peptides of the present invention. Said association of modified peptides can be linear or cyclized.

In a preferred embodiment, the peptide of the invention may comprise, or consist of the amino acid sequence of SEQ ID NO:1 wherein Xaa is selected independently of one another from the group consisting of lysine (Lys or K), histidine (His or H) and arginine (Arg or R) amino acid. In a preferred embodiment, Xaa represents either only lysine (Lys or K) residues (as shown in the amino acid sequence of SEQ ID NO:2) or arginine (Arg or R) residues (as shown in the amino acid sequence of SEQ ID NO:6).

In another preferred embodiment, the peptide of the invention may comprise, or consist of the amino acid sequence selected among the followings :

ppTG21 (20-mer) (SEQ ID NO :7)
Gly-Leu-Phe-His-Ala-Leu-Leu-His-Leu-Leu-His-Ser-Leu-Trp-
His-Leu-Leu-Leu-His-Ala

ppTG22 (21-mer) (SEQ ID NO :8)
Gly-Leu-Phe-Lys-Ala-Leu-Leu-Lys-Leu-Leu-Lys-Ser-Leu-Trp-
Lys-Leu-Leu-Leu-Lys-Ala-Cys

ppTG23 (21-mer) (SEQ ID NO :9)
Cys-Gly-Leu-Phe-Lys-Ala-Leu-Leu-Lys-Leu-Leu-Lys-Ser-Leu-
Trp-Lys-Leu-Leu-Leu-Lys-Ala

ppTG24 (22-mer) (SEQ ID NO :10)
Cys-Gly-Leu-Phe-Lys-Ala-Leu-Leu-Lys-Leu-Leu-Lys-Ser-Leu-
Trp-Lys-Leu-Leu-Leu-Lys-Ala-Cys

ppTG25 (20-mer) (SEQ ID NO :11) ppTG1-linker-SV40NLS
Gly-Leu-Phe-Lys-Ala-Leu-Leu-Lys-Leu-Leu-Lys-Ser-Leu-Trp-
Lys-Leu-Leu-Leu-Lys-Ala-Gly-Gly-Gly-Pro-Lys-Lys-Lys-Arg-
Lys-Val-Glu-Asp

ppTG26   (20-mer)   (SEQ   ID   NO :12)ppTG1-linker-SV40NLSm
(mutated)
Gly-Leu-Phe-Lys-Ala-Leu-Leu-Lys-Leu-Leu-Lys-Ser-Leu-Trp-
Lys-Leu-Leu-Leu-Lys-Ala-Gly-Gly-Gly-Pro-Lys-Thr-Lys-Arg-
Lys-Val-Glu-Asp

ppTG27 (20-mer) (SEQ ID NO :13) ppTG1-linker-SV40NLSrev
(reversed)
Gly-Leu-Phe-Lys-Ala-Leu-Leu-Lys-Leu-Leu-Lys-Ser-Leu-Trp-
Lys-Leu-Leu-Leu-Lys-Ala-Gly-Gly-Gly-Asp-Glu-Val-Lys-Arg-
Lys-Lys-Lys-Pro

ppTG28 (20-mer) (SEQ ID NO :14)
Gly-Leu-Phe-Lys-Lys-Leu-Leu-Lys-Leu-Leu-Lys-Lys-Leu-Trp-
Lys-Leu-Leu-Leu-Lys-Ala

ppTG29 (20-mer) (SEQ ID NO :15)
Gly-Leu-Phe-Arg-Arg-Leu-Leu-Arg-Leu-Leu-Arg-Arg-Leu-Trp-
Arg-Leu-Leu-Leu-Arg-Ala

ppTG30 (20-mer) (SEQ ID NO :16)
Gly-Ile-Phe-Lys-Ala-Ile-Ile-Lys-Ile-Ile-Lys-Ser-Ile-Trp-
Lys-Ile-Ile-Ile-Lys-Ala

ppTG31 (20-mer) (SEQ ID NO :17)
Gly-Ile-Phe-Arg-Ala-Ile-Ile-Arg-Ile-Ile-Arg-Ser-Ile-Trp-
Arg-Ile-Ile-Ile-Arg-Ala

ppTG32 (20-mer) (SEQ ID NO :18)
Gly-Val-Phe-Lys-Ala-Val-Val-Lys-Val-Val-Lys-Ser-Val-Trp-
Lys-Val-Val-Val-Lys-Ala

ppTG33 (20-mer) (SEQ ID NO :19)
Gly-Val-Phe-Arg-Ala-Val-Val-Arg-Val-Val-Arg-Ser-Val-Trp-
Arg-Val-Val-Val-Arg-Ala

ppTG20-D-configuration (20-mer) (SEQ ID NO :20)
Gly-Leu-Phe-Arg-Ala-Leu-Leu-Arg-Leu-Leu-Arg-Ser-Leu-Trp-
Arg-Leu-Leu-Leu-Arg-Ala

In another aspect the invention provides a complex for transferring an anionic substance of interest into a cell comprising:
(i) at least one peptide of the present invention,
(ii) at least one anionic substance of interest.

"Anionic substance of interest" designates a negatively-charged molecule without a limitation of the number of charges. Preferably, said molecule can be selected from the group consisting of proteins and nucleic acid molecules. According to a preferred embodiment, said anionic substance of interest is a nucleic acid molecule.

The term "nucleic acid" or "nucleic acid molecule" as used in the scope of the present invention means a DNA or RNA or a fragment or combination thereof, which is single-or double-stranded, linear or circular, natural or synthetic, modified or not (see US 5525711, US 4711955, US 5792608 or EP 302 175 for modification examples) without size limitation. It may, *inter alia*, be a genomic DNA, a cDNA, an mRNA, an antisense RNA, a ribozyme, or a DNA encoding such RNAs. The terms "polynucleotide", "nucleic acid molecule" and "nucleic acids" are synonyms with regard to the present invention. The nucleic acid may be in the form of a linear or circular polynucleotide, and preferably in the form of a plasmid. The nucleic acid can also be an oligonucleotide which is to be delivered to the cell, e.g., for antisense or ribozyme functions. According to the invention, the nucleic acid is preferably a naked polynucleotide (Wolff et al., Science 247 (1990), 1465-1468) or is formulated with at least one compound such as a polypeptide, preferably a viral polypeptide, or a cationic lipid, or a cationic polymer, or combination thereof, which can participate in the uptake of the polynucleotide into the cells (see Ledley, Human Gene Therapy 6 (1995), 1129-1144 for a review) or a protic polar compound (examples are provided below in the present application or in EP 890362).

Preferably, said nucleic acid molecule includes at least one encoding gene sequence of interest (i.e. a transcriptional unit) that can be transcribed and translated to generate a polypeptide of interest and the elements enabling its expression (i.e. an expression cassette). If the nucleic acid contains this proper genetic information when it is placed in an environment suitable for gene expression, its transcriptional unit will thus express the encoded gene product. The level and cell specificity of expression will depend to a significant extent on the strength and origin of the associated promoter and the presence and activation of an associated enhancer element. Thus in a preferred embodiment, the transcriptional control element include the promoter/enhancer sequences such as the CMV promoter/enhancer. However, those skilled in the art will recognize that a variety of other promoter and/or enhancer sequences are known which may be obtained from any viral, prokaryotic, e.g. bacterial, or eukaryotic organism, which are constitutive or regulable, which are suitable for expression in eukaryotic cells, and particularly in target cells. More precisely, this genetic information necessary for expression by a target cell comprises all the elements required for transcription of said gene sequence (if this gene sequence is DNA) into RNA, preferably into mRNA, and, if necessary, for translation of the mRNA into a polypeptide. Promoters suitable for use in various vertebrate systems are widely described in literature. Suitable promoters include but are not limited to the adenoviral E1a, MLP, PGK (Phospho Glycero Kinase ; Adra et al. Gene 60 (1987) 65-74 ; Hitzman et al. Science 219 (1983) 620-625), RSV, MPSV, SV40, CMV or 7.5k, the vaccinia promoter, inducible promoters, MT (metallothioneine; Mc Ivor et al., Mol. Cell Biol. 7 (1987), 838-848), alpha-1 antitrypsin, CFTR, immunoglobulin, alpha-actin (Tabin et al., Mol. Cell Biol. 2 (1982), 426-436), SR (Takebe et al., Mol. Cell. Biol. 8 (1988), 466-472), early SV40 (Simian Virus), RSV (Rous Sarcoma Virus) LTR, TK-HSV-1, SM22 (WO 97/38974), Desmin (WO 96/26284) and early CMV (Cytomegalovirus ; Boshart et al. Cell 41 (1985) 521), etc. Alternatively, one may use a synthetic promoter such as those described in Chakrabarti et al. (1997, Biotechniques 23, 1094-1097), Hammond et al. (1997, J. Virological Methods 66, 135-138) or Kumar and Boyle (1990, Virology 179, 151-158) as well as chimeric promoters between early and late poxviral promoters. Alternatively, promoters can be used which are active in tumor cells. Suitable examples include but are not limited to the promoters isolated from the gene encoding a protein selected from the group consisting of MUC-1 (overexpressed in breast and prostate cancers ; Chen et al., J. Clin. Invest. 96 (1995), 2775-2782), CEA (Carcinoma Embryonic Antigen ; overexpressed in colon cancers ; Schrewe et al., Mol, Cell. Biol. 10 (1990), 2738-2748), tyrosinase (overexpressed in melanomas ; Vile et al., Cancer Res. 53 (1993), 3860-3864), ErbB-2 (overexpressed in breast and pancreas cancers ; Harris et al., Gene Therapy 1 (1994), 170-175) and alpha-foetoprotein (overexpressed in liver cancers ; Kanai et al., Cancer Res. 57 (1997), 461-465) or combinations thereof. The early CMV promoter is preferred in the context of the invention.

The nucleic acid can also include intron sequences, targeting sequences, transport sequences, sequences involved in replication or integration. Said sequences have been reported in the literature and can readily be obtained by those skilled in the art. The nucleic acid can also be modified in order to be stabilized with specific components, for example spermine. It can also be substituted, for example by chemical modification, in order to facilitate its binding with specific polypeptides such as, for example the peptides of the present invention. According to the invention, the nucleic acid can be homologous or heterologous to the target cells into which it is introduced.

In a preferred embodiment, the nucleic acid contains at least one gene sequence of interest encoding a gene product which is a therapeutic molecule (i.e. a therapeutic gene). A "therapeutic molecule" is one which has a pharmacological or protective activity when administered appropriately to a patient, especially patient suffering from a disease or illness condition or who should be protected against this disease or condition. Such a pharmacological or protective activity is one which is expected to be related to a beneficial effect on the course or a symptom of said disease or said condition. When the skilled man selects in the course of applying the present invention a gene encoding a therapeutic molecule, he generally relates his choice to results previously obtained and can reasonably expect, without undue experiment other than practicing the invention as claimed, to obtain such pharmacological property. According to the invention, the sequence of interest can be homologous or heterologous to the target cells into which it is introduced. Advantageously said sequence of interest encodes all or part of a polypeptide, especially a therapeutic or prophylactic polypeptide giving a therapeutic or prophylactic effect. A polypeptide is understood to be any translational product of a polynucleotide regardless of size, and whether glycosylated or not, and includes peptides and proteins. Therapeutic polypeptides include as a primary example those polypeptides that can compensate for defective or deficient proteins in an animal or human organism, or those that act through toxic effects to limit or remove harmful cells from the body. They can also be immunity conferring polypeptides which act as an endogenous antigen to provoke a humoral or cellular response, or both.

The following encoding gene sequences are of particular interest. For example genes coding for a cytokine (α,β or γ-interferon, interleukine (IL), in particular IL-2, IL-6, IL-10 or IL-12, a tumor necrosis factor (TNF), a colony stimulating factor (such as GM-CSF, C-CSF, M-CSF), an immunostimulatory polypeptide (such as B7.1, B7.2, CD40, CD4, CD8, ICAM and the like), a cell or nuclear receptor, a receptor ligand (such as fas ligand), a coagulation factor (such as FVIII, FIX), a growth factor (such as Transforming Growth Factor TGF, Fibroblast Growth Factor FGF and the like), an enzyme (such as urease, renin, thrombin, metalloproteinase, nitric oxide synthase NOS, SOD, catalase), an enzyme inhibitor (such as α1-antitrypsine, antithrombine III, viral protease inhibitor, plasminogen activator inhibitor PAI-1), the CFTR protein, insulin, dystrophin, aa MHC antigen (Major Histocompatibility Complex) of class I or II or a polypeptide that can modulate/regulate the expression of one or more cellular genes, a polypeptide capable of inhibiting a bacterial, parasitic or viral infection or its development (such as antigenic polypeptides, antigenic epitopes, transdominant variants inhibiting the action of a native protein by competition), an apoptosis inducer or inhibitor (such as Bax, Bcl2, BclX), a cytostatic agent (such as p21, p16, Rb), an apolipoprotein (such as ApoAI, ApoAIV, ApoE), an inhibitor of angiogenesis (such as angiostatin, endostatin), an angiogenic polypeptide (such as family of Vascular Endothelial Growth Factors VEGF, FGF family, CCN family including CTGF, Cyr61 and Nov), an oxygen radical scavenger, a polypeptide having an anti-tumor effect, an antibody, a toxin, an immunotoxin and a marker (such as beta-galactosidase, luciferase) or any other gene of interest that is recognized in the art as being useful for the treatment or prevention of a clinical condition. In view of treating a hereditary dysfunction, one may use a functional allele of a defective gene, for example a gene encoding factor VIII or IX in the context of haemophilia A or B, dystrophin (or minidystrophin) in the context of myopathies, insulin in the context of diabetes, CFTR (Cystic Fibrosis Transmembrane Conductance Regulator) in the context of cystic fibrosis. Suitable anti-tumor genes include but are not limited to those encoding an antisense RNA, a ribozyme, a cytotoxic product such as thymidine kinase of herpes-1 simplex virus (TK-HSV-1), ricin, a bacterial toxin, the expression product of yeast genes *FCY1* and/or *FUR1* having UPRTase (Uracile Phosphoribosyltransferase) and CDase (Cytosine Deaminase) activity respectively, an antibody, a polypeptide inhibiting cellular division or transduction signals, a tumor suppressor gene (p53, Rb, p73), a polypeptide activating host immune system, a tumor-associated antigen (MUC-1, BRCA-1, an HPV early or late antigen (E6, E7, L1, L2), optionally in combination with a cytokine gene. The polynucleotide can also encode an antibody. In this regard, the term "antibody" encompasses whole immunoglobulins of any class, chimeric antibodies and hybrid antibodies with dual or multiple antigen or epitope specificities, and fragments, such as F(ab)'₂, Fab', Fab including hybrid fragments and anti-idiotypes (US 4,699,880). Advantageously said nucleic acid encodes all or part of a polypeptide which is an immunity conferring polypeptide and acts as endogenous immunogen to provoke a humoral or cellular response, or both, against infectious agents, including intracellular viruses, or against tumor cells. An "immunity-conferring polypeptide" means that said polypeptide when it is produced in the transfected cells will participate in an immune response in the treated patient. More specifically, said polypeptide produced in or taken up by macropinocyte cells such as APCs will be processed and the resulting fragments will be presented on the surface of these cells by MHC class I and/or II molecules in order to elicit a specific immune response.

The nucleic acid may comprise one or more gene(s) of interest. In this regard, the combination of genes encoding a suicide gene product and a cytokine gene (e.g. α, β or γ interferons, interleukins, preferably selected among IL-2,IL-4,IL-6, IL-10 or IL-12, TNF factors, GM-CSF, C-CSF, M-CSF and the like), an immunostimulatory gene (e.g. B7.1, B7.2, ICAM) or a chimiokine gene (e.g. MIP , RANTES, MCP 1) is advantageous. The different gene expression may be controlled by a unique promoter (polycistronic cassette) or by independent promoters. Moreover, they may be inserted in a unique site or in various sites along the nucleic acid either in the same or opposite directions.

The encoding gene sequence of interest may be isolated from any organism or cell by conventional techniques of molecular biology (PCR, cloning with appropriate probes, chemical synthesis) and if needed its sequence may be modified by mutagenesis, PCR or any other protocol.

The introduction or transfer process of an anionic substance of interest into a cell is by itself well known. "Introduction or transfer" means that the anionic substance is transferred into the cell and is located, at the end of the process, inside said cell or within or on its membrane. If the anionic substance is a nucleic acid, "introduction or transfer" is also referred to as "transfection". Transfection can be verified by any appropriate method, for example by measuring the expression of a gene encoded by said nucleic acid or by measuring the concentration of the expressed protein or its mRNA, or by measuring its biological effect.

"Capable of binding to" means that the considered compound is capable to interact and to bind to another compound, preferably in a reversible manner, for example by ionic interactions, by forming disulfide or hydrogen bonds, by hydrophobic interactions or covalent bonds. According to a particular embodiment, a peptide of the invention is capable of interacting and binding to an anionic substance of interest, preferably to a single stranded and/or double stranded nucleic acid. Preferably, a peptide of the invention is capable of interacting and binding to an anionic substance of interest at least by the intermediate of ionic interactions. Accordingly, the term "complex" refers to molecular assemblages of at least one peptide of the present invention and at least one anionic substance which are bound to one another via any one of the above described binding activities. Such a complex may contain further elements which are described in the follow.

According to a particular embodiment, the complex of the invention further comprises :
(iii) at least one ligand capable of cell-specific and/or nuclear targeting ; and/or
(iv) at least one further peptide which is capable of causing membrane disruption ; and/or
(v) at least one cationic compound selected from the group consisting of cationic lipids and cationic polymers and/or
(vi) at least one colipid.

The term "ligand capable of cell-specific targeting" refers to a ligand moiety which binds to a surface receptor of a cellular membrane (i.e. anti-ligand). Said cell membrane surface receptor is a molecule or structure which can bind said ligand with high affinity and preferably with high specificity. Said cell membrane surface receptor is preferably specific for a particular cell, i.e. it is found predominantly in one type of cells rather than in another type of cells (e.g. galactosyl residues to target the asialoglycoprotein receptor on the surface of hepatocytes). The cell membrane surface receptor facilitates cell targeting and internalization into the target cell of the ligand (i.e. the peptide involved in cell-specific targeting) and attached molecules (i.e. the complex of the invention).

A large number of ligand moieties /anti-ligands that may be used in the context of the present invention are widely described in the literature. Such a ligand moiety is capable of conferring to the complex of the invention, the ability to bind to a given anti-ligand molecule or a class of anti-ligand molecules localized at the surface of at least one target cell. Suitable anti-ligand molecules include without limitation polypeptides selected from the group consisting of cell-specific markers, tissue-specific markers, cellular receptors, viral antigens, antigenic epitopes and tumor-associated markers, Anti-ligand molecules may moreover consist of or comprise one or more sugar, lipid, glycolipid or antibody molecules. According to the invention, a ligand moiety may be for example a lipid, a glycolipid, a hormone, a sugar, a polymer (e.g. PEG, polylysine, PEI), an oligonucleotide, a vitamin, an antigen, all or part of a lectin, all or part of a polypeptide such as for example JTS1 (WO 94/40958), an antibody or a fragment thereof, or a combination thereof.

Preferably, the ligand moiety used in the present invention is a peptide or polypeptide having a minimal length of 7 amino acids. It is either a native polypeptide or a polypeptide derived from a native polypeptide. "Derived" means containing (a) one or more modifications with respect to the native sequence (e.g. addition, deletion and/or substitution of one or more residues), (b) amino acid analogs, including not naturally occurring amino acids or (c) substituted linkages or (d) other modifications known in the art. The polypeptides serving as ligand moiety encompass variant and chimeric polypeptides obtained by fusing sequences of various origins, such as for example a humanized antibody which combines the variable region of a mouse antibody and the constant region of a human immunoglobulin. In addition, such polypeptides may have a linear or cyclized structure (e.g. by flanking at both extremities a polypeptide ligand by cysteine residues). Additionally, the polypeptide in use as ligand moiety may include modifications of its original structure by way of substitution or addition of chemical moieties (e.g. glycosylation, alkylation, acetylation, amidation, phosphorylation, addition of sulfhydryl groups and the like). The invention further contemplates modifications that render the ligand moiety detectable. For this purpose, modifications with a detectable moiety can be envisaged (i.e. a scintigraphic, radioactive, or fluorescent moiety, or a dye label and the like). Suitable radioactive labels include but are not limited to Tc^{99m}, I¹²³ and In¹¹¹. Such detectable labels may be attached to the ligand moiety by any conventional techniques and may be used for diagnostic purposes (e.g. imaging of tumoral cells).

In one special embodiment, the anti-ligand molecule is an antigen (e.g. a target cell-specific antigen, a disease-specific antigen, an antigen specifically expressed on the surface of engineered target cells) and the ligand moiety is an antibody, a fragment or a minimal recognition unit thereof ( i.e. a fragment still presenting an antigenic specificity) such as those described in detail in immunology manuals (see for example Immunology, third edition 1993, Roitt, Brostoff and Male, ed Gambli, Mosby). The ligand moiety may be a monoclonal antibody. Monoclonal antibodies which will bind to many of these antigens are already known but in any case, with today's techniques in relation to monoclonal antibody technology, antibodies may be prepared to most antigens. The ligand moiety may be a part of an antibody (for example a Fab fragment) or a synthetic antibody fragment (for example, ScFv).

Suitable monoclonal antibodies to selected antigens may be prepared by known techniques, for example those disclosed in "Monoclonal Antibodies: A manual of techniques", H. Zola (CRC Press, 1988) and in "Monoclonal Hybridoma Antibodies: Techniques and Applications", J. G. R. Hurrell (CRC Press, 1982). Suitably prepared non-human antibodies may be "humanized" in known ways, for example by inserting the CDR regions of mouse antibodies into the framework of human antibodies. Additionally, as the variable heavy (VH) and variable light (VL) domains of the antibody are involved in antigen recognition, variable domains of rodent origin may be fused to constant domains of human origin such that the resultant antibody retains the antigenic specificity of the rodent parental antibody (Morrison et al (1984) Proc. Natl. Acad. Sci. USA 81, 6851-6855).

Antigenic specificity of antibodies is conferred by variable domains including Fab-like molecules (Better et al (1988) Science 240, 1041); Fv molecules (Skerra et al (1988) Science 240, 1038); ScFv molecules where the VH and VL partner domains are linked via a flexible oligopeptide (Bird et al (1988) Science 242, 423; Huston et al (1988) Proc. Natl. Acad. Sci. USA 85, 5879) and dAbs comprising isolated V domains (Ward et al (1989) Nature 341, 544). A general review of the techniques involved in the synthesis of antibody fragments which retain their specific binding sites is to be found in Winter & Milstein (1991) Nature 349, 293-299.

According to an advantageous embodiment, the ligand moiety is selected among antibody fragments, rather than whole antibodies. Effective functions of whole antibodies, such as complement binding, are removed. ScFv and dAb antibody fragments may be expressed as a fusion with one or more other polypeptides. Minimal recognition units may be derived from the sequence of one or more of the complementary-determining regions (CDR) of the Fv fragment. Whole antibodies, and F(ab')2 fragments are "bivalent". By "bivalent" we mean that said antibodies and F(ab') 2 fragments have two antigen binding sites. In contrast, Fab, Fv, ScFv, dAb fragments and minimal recognition units are monovalent, having only one antigen binding sites.

In a further embodiment the ligand moiety is at least part of a specific moiety implicated in natural cell-surface receptor binding. Of course, said natural receptors (e.g. hormone receptors) may also be target cell-specific antigens and may be recognized by ligand moieties which have the property of a monoclonal antibody, a ScFv, a dAb or a minimal recognition unit.

In a preferred embodiment, the ligand moiety allows to target a virally infected cell and is capable of recognizing and binding to a viral component (e.g. envelope glycoprotein) or capable of interfering with the virus biology (e.g. entry or replication). For example, the targeting of an HIV (Human Immunodeficiency Virus) infected cell can be performed with a ligand moiety specific for an epitope of the HIV envelope, such as a ligand moiety derived from the 2F5 antibody (Buchacher et al., 1992, Vaccines 92, 191-195) recognizing a highly conserved epitope of the transmembrane glycoprotein gp41 or with a ligand moiety interfering with HIV attachment to its cellular receptor CD4 (e.g. the extracellular domain of the CD4 molecule).

In another preferred embodiment, the ligand moiety allows to target a tumor cell and is capable of recognizing and binding to a molecule related to the tumor status, such as a tumor-specific antigen, a cellular protein differentially or over-expressed in tumor cells or a gene product of a cancer-associated virus.

Examples of tumor-specific antigens include but are not limited to MUC-1 related to breast cancer (Hareuveni et al., 1990, Eur. J. Biochem 189, 475-486), the products encoded by the mutated *BRCA*1 and *BRCA*2 genes related to breast and ovarian cancers (Miki et al., 1994, Science 226, 66-71 ; Futreal et al., 1994, Science 226, 120-122 ; Wooster et al., 1995, Nature 378, 789-792), APC related to colon cancer (Polakis, 1995, Curr. Opin. Genet. Dev. 5, 66-71), prostate specific antigen (PSA) related to prostate cancer, (Stamey et al., 1987, New England J. Med. 317, 909), carcinoma embryonic antigen (CEA) related to colon cancers (Schrewe et al., 1990, Mol. Cell. Biol. 10, 2738-2748), tyrosinase related to melanomas (Vile et al., 1993, Cancer Res. 53, 3860-3864), receptor for melanocyte-stimulating hormone (MSH) which is expressed in high number in melanoma cells, ErbB-2 related to breast and pancreas cancers (Harris et al., 1994, Gene Therapy 1, 170-175), and alpha-foetoprotein related to liver cancers (Kanai et al., 1997, Cancer Res. 57, 461-465).

A special ligand moiety in use in the present invention is a fragment of an antibody capable of recognizing and binding to the MUC-1 antigen and thus targeting the MUC-1 positive tumor cells. A more preferred ligand moiety is the scFv fragment of the SM3 monoclonal antibody which recognizes the tandem repeat region of the MUC-1 antigen (Burshell et al., 1987, Cancer Res. 47, 5476-5482 ; Girling et al., 1989, Int J. Cancer 43, 1072-1076 ; Dokurno et al., 1998, J. Mol. Biol. 284, 713-728).

Examples of cellular proteins differentially or overexpressed in tumor cells include but are not limited to the receptor for interleukin 2 (IL-2) overexpressed in some lymphoid tumors, GRP (Gastrin Release Peptide) overexpressed in lung carcinoma cells, pancreas, prostate and stomach tumors (Michael et al., 1995, Gene Therapy 2, 660-668), TNF (Tumor Necrosis Factor) receptor, epidermal growth factor receptors, Fas receptor, CD40 receptor, CD30 receptor, CD27 receptor, OX-40, αv integrins (Brooks et al., 1994, Science 264, 569) and receptors for certain angiogenic growth factors (Hanahan, 1997, Science 277, 48). Based on these indications, it is within the scope of those skilled in the art to define an appropriate ligand moiety capable of recognizing and binding to such proteins. To illustrate, IL-2 is a suitable ligand moiety to bind to IL-2 receptor.

Suitable gene products of cancer-associated viruses include but are not limited to human papilloma virus (HPV) E6 and E7 early polypeptides as well as L1 and L2 late polypeptides (EP 0 462 187, US 5,744,133 and WO98/04705) that are expressed in cervical cancer and EBNA-1 antigen of Epstein-Barr virus (EBV) associated with Burkitt's lymphomas (Evans et al., 1997, Gene Therapy 4, 264-267).

In still another embodiment, the ligand moiety allows to target tissue-specific molecules. For example, ligand moieties suitable for targeting liver cells include but are not limited to those derived from ApoB (apolipoprotein) capable of binding to the LDL receptor, alpha-2-macroglobulin capable of binding to the LPR receptor, alpha-1 acid glycoprotein capable of binding to the asialoglycoprotein receptor and transferrin capable of binding to the transferrin receptor. A ligand moiety for targeting activated endothelial cells may be derived from the sialyl-Lewis-X antigen (capable of binding to ELAM-1), from VLA-4 (capable of binding to the VCAM-1 receptor) or from LFA-1 (capable of binding to the ICAM-1 receptor). A ligand moiety derived from CD34 is useful to target hematopoïetic progenitor cells through binding to the CD34 receptor. A ligand moiety derived from ICAM-1 is more intended to target lymphocytes through binding to the LFA-1 receptor. Finally, the targeting of T-helper cells may use a ligand moiety derived from HIV gp-120 or a class II MHC antigen capable of binding to the CD4 receptor.

By "target cells" , we refer to the cells that the complex of the invention can selectively target. Depending on the nature of the ligand moiety and/or of the anti-ligand molecule, "target cells" may designate a unique type of cell or a group of different types of cells having as a common feature on their surface an anti-ligand molecule(s) recognized by ligand moiety(s) present in the complex of the invention. For the purpose of the invention, a target cell is any mammalian cell (preferably human cell) which can be targeted with a complex according to the present invention having a suitable ligand moiety. The term "to target" refers to addressing a certain type of cells or a group of types of cells for gene transfer in favour of the remaining part of the totality of cells being contacted with the complex of the present invention. The target cell may be a primary cell, a transformed cell or a tumor cell. Suitable target cells include but are not limited to hematopoïetic cells (totipotent, stem cells, leukocytes, lymphocytes, monocytes, macrophages, APC, dendritic cells , non-human cells and the like), muscle cells (satellite, myocytes, myoblasts, skeletal or smooth muscle cells, heart cells), pulmonary cells , tracheal cells, hepatic cells, epithelial cells, endothelial cells or fibroblasts.

The term "ligand capable of nuclear targeting" refers to a particular ligand which is capable of binding to a nuclear receptor (nuclear anti-ligand). Said nuclear receptor is a molecule or structure localized in or/and on the nuclear membrane which can bind to said ligand, thereby facilitating intracellular transport of the complex of the present invention towards the nucleus and its internalization into the nucleus. Examples of such a ligand involved in nuclear targeting are the nuclear signal sequences derived from the T-antigen of the SV40 virus (Lanford and Butel, 1984, Cell 37, 801-813) and from the EBNA-1 protein of the Epstein Barr virus (Ambinder et al., 1991, J. Virol. 65, 1466-1478).

In a special embodiment, the complex of the invention may comprise (iv) at least one further peptide capable of causing membrane disruption. Contrary to the peptide of the instant invention, said second peptide is not necessarily a cationic peptide capable of binding with anionic molecules. Examples of such peptides are JTS-1, JTS-1-K13, GALA, KALA (see Mahato et al., 1999, Current Opinion in Mol. Therapeutics 1, 226-243; WO 96/40958 ; WO 98/50078 ; Gottschalk et al., 1996, Gene Therapy, 3, 448-457 Haensler & Szoka, 1993, Bioconjugate Chem., 4, 372-379 ; Wyman et al., 1997, Biochemistry, 36, 3008-3017).

In another embodiment, the complex of the invention may further comprise (v) at least one cationic compound selected from the group consisting of cationic lipids and cationic polymers. These cationic compounds are widely described in the scientific literature (see for example the references related to non-viral delivery systems mentioned above, or WO 97/29118, WO 98/08489, WO 98/17693). Cationic lipids or mixtures of cationic lipids which may be used in the present invention include cationic lipids selected from the group consisting of Lipofectin™, DOTMA: N-[1-(2,3-dioleyloxyl)propyl]-N,N,N-trimethylammonium (Felgner, PNAS 84 (1987), 7413-7417), DOGS: dioctadecylamidoglycylspermine or Transfectam™ (Behr, PNAS 86 (1989), 6982-6986), DMRIE: 1,2-dimiristyloxypropyl-3-dimethyl-hydroxyethylammonium and DORIE: 1,2-diooleyloxypropyl-3-dimethyl-hydroxyethylammnoium (Felgner, Methods 5 (1993), 67-75), DC-CHOL: 3 [N-(N',N'-dimethylaminoethane)-carbamoyl]cholesterol (Gao, BBRC 179 (1991), 280-285), DOTAP (McLachlan, Gene Therapy 2 (1995), 674-622), Lipofectamine™, spermine or spermidine-cholesterol, Lipofectace™ (for a review see for example Legendre, Medecine/Science 12 (1996), 1334-1341 or Gao, Gene Therapy 2 (1995), 710-722), cationic lipid as disclosed in patent applications WO 98/34910, WO 98/14439, WO 97/19675, WO 97/37966 and their isomers. Nevertheless, this list is not exhaustive and other cationic lipids well known in the art can be used in connection with the present invention as well.

Preferably, the cationic lipids of the present invention are selected from the cationic lipids having the formula (EP 901 463):

CH₂-O-R₁

CH-O-R₂

CH₂-X-CO-CH₂-(-NH-(CH₂)ₘ-)ₙ-NH₂

wherein :
R₁, R₂, are identical or different and are C₆-C₂₃ alkyl or alkenyl, linear or branched, or -C(=O)-(C₆-C₂₃)alkyl or -C(=O)-(C₆-C₂₃)alkenyl, linear or branched,
X is O or -NR₃, R₃ being H or C₁-C₄ alkyl,
n = 1 to 6,
m = 1 to 6, and when n > 1, m can be identical or different in each n repeat.

Cationic polymers or mixtures of cationic polymers which may be used in the present invention include cationic polymers selected from the group consisting of chitosan, poly(aminoacids) such as polylysine (US-A-5,595,897 and FR 2 719 316); polyquaternary compounds; protamine; polyimines;polyethylene imine or polypropylene imine (WO 96/02655) ; polyvinylamines; polycationic polymer derivatized with DEAF, such as pullulans, celluloses; polyvinylpyridine; polymethacrylates; polyacrylates; polyoxethanes; polythiodiethylaminomethylethylene (P(TDAE)); polyhistidine; polyornithine; poly-p-aminostyrene; polyoxethanes; co-polymethacrylates (eg copolymer of HPMA; N-(2-hydroxypropyl)-methacrylamide); the compound disclosed in US-A-3,910,862, polyvinylpyrrolid complexes of DEAE with methacrylate, dextran, acrylamide, polyimines, albumin, onedimethylaminomethylmethacrylates and polyvinylpyrrolidonemethylacrylaminopropyltrimethyl ammonium chlorides; polyamidoamines; telomeric compounds . Nevertheless, this list is not exhaustive and other cationic polymers well known in the art can be used in connection with the present invention as well.

Colipids (vi) may be optionally included in the complex of the invention in order to facilitate entry of the nucleic acid into the cell. According to the invention, colipids are selected from the group consisting of positively or negatively charged, neutral or zwitterionic lipids. These colipids are, for example, selected from the group consisting of phosphatidylethanolamine (PE), phosphatidylcholine, phosphocholine, dioleylphosphatidylethanolamine (DOPE), sphingomyelin, ceramide or cerebroside and one of their derivatives.

The various elements of the complex (i.e. ligand, peptide, anionic or cationic compounds) may be modified or substituted by chemical or natural processes widely used by the skilled man in order to obtain compounds modified or substituted such as those disclosed above, enabling, for example, visualization of the distribution of the polypeptide expressed by the nucleic acid, of the nucleic acid, or of the complex of the invention, after *in vitro* or *in vivo* administration of the complex.

In a specific embodiment of the invention, the size of the complex according to the invention is small (i.e. its diameter is less than 2*µ*m, preferably less than 500 nm and, most preferably, it ranges between 20 and 100 nm). The size of the complex may be selected for optimal use in particular applications. Measurements of the complex size can be achieved by a number of techniques including, but not limited to, dynamic laser light scattering (photon correlation spectroscopy, PCS), as well as other techniques known to those skilled in the art (see, Washington, Particle Size Analysis in Pharmaceutics and other Industries, Ellis Horwood, New York, 1992, 135-169). Sizing procedure may also be applied on complexes in order to select specific complex sizes. Methods which can be used in this sizing step include, but are not limited to, extrusion, sonication and microfluidization, size exclusion chromatography, field flow fractionation, electrophoresis and ultracentrifugation.

As a preferred embodiment of the invention, the ratio between the number of positive charges and the number of negative charges of the complex is between 0,05 and 20, preferably said ratio is up to 1. The complex of the invention may be characterized by its theoretical charge ratio (+/-), which is the ratio of the positive charges provided by at least the peptide of the invention (i) to the negative charges provided by at least the anionic substance of interest (ii) in the complex, assuming that all potentially cationic groups are in fact in the cationic state and all potentially anionic groups are in fact in the anionic state. In general, an excess of positive charges on the complex facilitates binding of the complex to the negatively-charged cell surface. To obtain such a ratio, the calculation shall take into account all negative charges in the anionic substance and shall then adjust the quantity of the peptide of the present invention necessary to obtain the desired theoretical charge ratio as defined above. The quantities and the concentrations of the other ingredients (iii)-(vi), if any, shall be taken into account in function of their respective molar masses and their number of positive and negative charges. The ratio is not specifically limited. As a preferred embodiment the above identiified quantities are selected so that the ratio between the number of positive charges and the number of negative charges is between 0.05 and 20, preferably said charge ratio is up to 1.

The ratio of cationic lipids and/or cationic polymers to colipid (on a weight to weight basis), when the compounds are co-existing in the complex, can range from 1:0 to 1:10. In preferred embodiments, this ratio ranges from 1:0.5 to 1:4.

The compound and charge ratios indicated herein are not meant to be limiting as one skilled in the art could readily practice the teachings of the invention using any ratio of the herein disclosed components.

Furthermore, the concentration of the anionic substance of interest (ii) which may be added to the peptide to form said complex of the invention may range from 10 *µ*g/ml to 5000 *µ*g/ml. In a preferred embodiment of the invention, the concentration of said anionic substance of interest ranges from 100 *µ*g/ml to 1000 *µ*g/ml. Doses based on a plasmid or synthetic vector may comprise between 0.01 and 100 mg of DNA, advantageously between 0.05 and 10 mg and preferably between 0.5 and 5 mg.

The invention is also directed to a process for the preparation of the above described complex, comprising the following steps:
- contacting at least one peptide capable of binding to an anionic substance of interest and which is a cationic peptide capable of causing membrane disruption and which does not comprise acidic amino acid, preferably which does not comprise a glutamic amino acid (i.e. a peptide of the present invention) with at least one anionic substance of interest,
- and recovering said complex, optionally after a purification or selecion step.

Where the complex of the invention further comprises:
(iii) at least one ligand capable of cell-specific and/or nuclear targeting ; and/or
(iv) at least one further peptide which is capable of causing membrane disruption ; and/or
(v) at least one cationic compound selected from the group consisting of cationic lipids and cationic polymers ; and/or
(vi) at least one colipid.
said process comprises the steps of:
- first mixing said peptide (i) with said additional element (iii) and/or (iv) and/or (v) and/or (vi) and then complexing the anionic substances of interest, or
- first complexing said peptide (i) with the anionic substances of interest (ii) and then mixing the complex with said additional element (iii) and/or (iv) and/or (v) and/or (vi).

The process can further comprise a sizing procedure as described above.

The invention also encompasses a composition, preferably for transferring an anionic substance of interest into a cell, wherein said composition comprises at least one complex as previously disclosed. Said composition is particularly useful for the delivery of polynucleotides to cells or tissues of a subject in connection with gene therapy methods but are not limited to such uses. The term "gene therapy method" is preferably understood as a method for the introduction of a polynucleotide into cells either *in vivo* or by introduction into cells *in vitro* followed by re-implantation into a subject. "Gene therapy" in particular concerns the case where the gene product is expressed in a tissue as well as the case where the gene product is excreted, especially into the blood stream.

This composition can be formulated in various forms, e.g. in solid, liquid, powder, aqueous, lyophilized form. In a preferred embodiment, this composition further comprises a pharmaceutically acceptable carrier, allowing its use in a method for the therapeutic treatment of humans or animals. In this particular case, the carrier is preferably a pharmaceutically suitable injectable carrier or diluent (for examples, see Remington's Pharmaceutical Sciences, 16^{th} ed. 1980, Mack Publishing Co). Such a carrier or diluent is pharmaceutically acceptable, i.e. is non-toxic to a recipient at the dosage and concentration employed. It is preferably isotonic, hypotonic or weakly hypertonic and has a relatively low ionic strength, such as provided by a sucrose solution. Furthermore, it may contain any relevant solvents, aqueous or partly aqueous liquid carriers comprising sterile, pyrogen-free water, dispersion media, coatings, and equivalents, or diluents (e.g. Tris-HCl, acetate, phosphate), emulsifiers, solubilizers or adjuvants. The pH of the pharmaceutical preparation is suitably adjusted and buffered in order to be useful in in vivo applications. It may be prepared either as a liquid solution or in a solid form (e.g. lyophilized) which can be suspended in a solution prior to administration. Representative examples of carriers or diluents for an injectable composition include water, isotonic saline solutions which are preferably buffered at a physiological pH (such as phosphate buffered saline or Tris buffered saline), mannitol, dextrose, glycerol and ethanol, as well as polypeptides or proteins such as human serum albumin. For example, such composition comprise 10 mg/ml mannitol, 1 mg/ml HSA, 20 mM Tris pH 7.2 and 150 mM NaCl.

The invention more particularly relates to a composition comprising at least one of the complexes described above and at least one adjuvant capable of improving the transfection capacity of said complex. Adjuvants may be selected from the group consisting of a chloroquine, protic polar compounds, such as propylene glycol, polyethylene glycol, glycerol, EtOH, 1-methyl L -2-pyrrolidone or their derivatives, or aprotic polar compounds such as dimethylsulfoxide (DMSO), diethylsulfoxide, di-n-propylsulfoxide, dimethylsulfone, sulfolane, dimethylformamide, dimethylacetamide, tetramethylurea, acetonitrile or their derivatives.

The composition of the present invention can be administered into a vertebrate tissue. This administration may be carried out by an intradermal, subdermal, intravenous, intramuscular, intranasal, intracerebral, intratracheal, intraarterial, intraperitoneal, intravesical, intrapleural, intracoronary or intratumoral injection, by means of a syringe or other devices. Transdermal administration is also contemplated, such as inhalation, aerosol routes, instillation or topical application. "Vertebrate" as used herein is intended to have the same meaning as commonly understood by one of ordinary skill in the art. Particularly, "vertebrate" encompasses mammals, and more particularly humans.

According to the present invention, the composition can be administered into tissues of the vertebrate body including those of muscle, skin, brain, lung, liver, spleen, bone marrow, thymus, heart, lymph, bone, cartilage, pancreas, kidney, gall bladder, stomach, intestine, testis, ovary, uterus, rectum, nervous system, eye, gland, connective tissue, blood, tumor, etc.

Applied to *in vivo* gene therapy, this invention allows repeated administration to the patient without any risk of the administered preparation to induce a significant immune reaction. Administration may be by single or repeated dose, once or several times after a certain period of time. Repeated administration allows a reduction of the dose of active substance, in particular DNA, administered at a single time. The route of administration and the appropriate dose varies depending on several parameters, for example the individual patient, the disease being treated, or the nucleic acid being transferred.

According to the invention, "cells" include prokaryotic cells and eukaryotic cells, yeast cells, plant cells, human or animal cells, in particular mammalian cells. In particular, cancer cells should be mentioned. The invention can be applied *in vivo* to the interstitial or luminal space of tissues in the lungs, the trachea, the skin, the muscles, the brain, the liver, the heart, the spleen, the bone marrow, the thymus, the bladder, the lymphatic system, the blood, the pancreas, the stomach, the kidneys, the ovaries, the testicles, the rectum, the peripheral or central nervous system, the eyes, the lymphoid organs, the cartilage, or the endothelium. In preferred embodiments, the cell will be a muscle cell, as stem cell of the hematopoietic system or an airways cell, more especially a tracheal or pulmonary cell, and preferably a cell of the respiratory epithelium.

The present invention also encompasses a process for transferring a nucleic acid into cells wherein said process comprises contacting said cells with at least one complex or composition according to the invention. This process may be applied by direct administration of said complex or composition to cells of the animal *in vivo,* or by *in vitro* treatment of cells which were recovered from the animal and then re-introduced into the animal body (*ex vivo* process). In *in vitro* applications, cells cultivated on an appropriate medium are placed in contact with a suspension containing a complex or composition of the invention. After an incubation time, the cells are washed and recovered. Introduction of the active substance can be verified (eventually after lysis of the cells) by any appropriate method.

In the case of *in vivo* treatment according to the invention, in order to improve the transfection rate, the patient may undergo a macrophage depletion treatment prior to administration of the pharmaceutical preparation as described above. Such a technique is described in the literature (refer particularly to Van Rooijen et al., 1997, TibTech, 15, 178-184).

Finally, the present invention also provides the use of a peptide according to the invention for the preparation of a pharmaceutical composition for curative, preventive or vaccine treatment of mammals. Preferably, such compositions are intended for gene transfer and more preferably for the treatment of the human or animal body by gene therapy. Within the meaning of the present invention, "gene therapy" has to be understood as a method for introducing any therapeutic gene into a cell. Thus, it also includes immunotherapy that relates to the introduction of a potentially antigenic epitope into a cell to induce an immune response which can be cellular or humoral or both. " Treatment" as used herein refers to prophylaxis and therapy. It concerns both the treatment of humans and animals. A " therapeutically effective amount of a peptide or a composition " is a dose sufficient for the alleviation of one or more symptoms normally associated with the disease desired to be treated. A method according to the invention is preferentially intended for the treatment of the diseases listed above.

The invention further concerns the use of a peptide or of a complex as defined above for the preparation of a composition for curative, preventive or vaccine treatment of man or animals, preferably mammals, and more specifically for gene therapy use.

The invention also concerns the use of a peptide of the invention for the preparation of a complex for transferring an anionic substance of interest into a cell. According to a preferred embodiment, said peptide does not comprise acidic amino acid, or even more preferred it does not comprise glutamic amino acid. In a preferred embodiment, said peptide has a molecular weight of less than 5 kD, most preferably of less than 3 kD. Preferably, said peptide consists of or comprises the amino acid sequence SEQ ID NO:1 wherein each Xaa is selected independently of one another from the group consisting of lysine (Lys or K), histidine (His or H) and arginine (Arg or R) residues. In a preferred embodiment, all Xaa represent either only lysine (Lys or K) residues (SEQ ID NO:2) or arginine (Arg or R) residues (SEQ ID NO:6).

These and other embodiments are disclosed or are obvious from and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on Internet, e.g. under http://www.ncbi.nlm.nih.gov/PubMed/medline.html. Further databases and addresses, such as http://www.ncbi.nlm.nih.gov, http://www.infobiogen.fr, http://www.fmi.ch/biology/research_tools.html, http://www.tigr.org, are known to the person skilled in the art and can also be obtained using, e.g., http://www.lycos.com. An overview of patent information in biotechnology and a survey of relevant sources of patent information useful for retrospective searching and for current awareness is given in Berks, TIBTECH 12 (1994), 352-364.

The methods, compositions and uses of the invention can be applied in the treatment of all kinds of diseases the treatment and/or diagnostic of which is related to or dependent on the transfer of nucleic acids in cells. The compositions, and uses of the present invention may be desirably employed in humans, although animal treatment is also encompassed by the uses described herein.

The invention has been described in an illustrative manner, and it is to be understood that the terminology which has been used is intended to be in the nature of words of description rather than of limitation. Obviously, many modifications and variations of the present invention are possible in light of the above teachings. It is therefore to be understood that within the scope of the appended claims, the invention may be practiced different from what is specifically described herein.

The disclosure of all patents, publications published patent applications, and database entries cited in the present application are hereby incorporated by reference in their entirety to the same extend as if each such individual patent, publication and database entry were specifically and individually indicated to be incorporated by reference and were set forth in its entirety herein.

### LEGENDS

Figure 1: Liposome leakage assay. Increasing amounts of the peptides JTS1, ppTG1, JTS1-K13, KALA and ppTG20 are indicated. Figure 1A shows the results of the liposome leakage assay carried out at pH5. Figure 1B summarizes the results obtained at pH7.

Figure 2: Transfection study in vitro - comparison of ppTG1, PEI and Lipofectin.293-EBNA cells were transfected with 0.5 µg, 0.1 µg, 0.05 µg or 0.01 µg of the plasmid pTG11056 formulated with Lipofectin, PEI or ppTG1 at the indicated charge ratio (+/-). Mock represents transfection with buffer.

Figure 3: Transfection study in vitro - comparison of ppTG1 and Lipofectin; effect of charge ratios. 7 x 10⁴ HeLa cells were plated on 24 well plates. The next day cells were transfected with 0.5 µg or 0.05 µg pTG11236 formulated with Lipofectin or ppTG1 at the indicated charge ratio.

Figure 4: Transfection study in vitro - comparison of ppTG1 and JTS1-K13. 5 x 10⁴ HeLa cells were transfected with 0.5 µg or 0.05 µg pTG11236 formulated with ppTG1 or JTS1-K13 at the indicated charge ratio. The calculations for JTS1-K13 are based on a net positive charge of +5 per molecule.

Figure 5: Transfection study in vitro - comparison of ppTG1 and KALA. 5 x 10⁴ HeLa or CHO cells were transfected with 50 or 500 ng pTG11236 (= p) formulated with ppTG1 or KALA at the indicated charge ratio. The luciferase activities determined 20h after transfection are indicated in Figure 5A (HeLa cells) and in Figure 5B (CHO cells).

Figure 6 : Transfection study in vitro -comparison of ppTG1 with and without pcTG90/DOPE. HeLa or CHO cells were transfected with 500ng and 50ng of pTG11236, complexed with ppTG1 or KALA. The charge ratio +/- varied from 1, 2, 3, 4, 7 to 10.

Figure 7: In vivo experiment.Five B6SJL mice per group were intravenously injected with 60 µg or 30 µg pTG11236 (=p) formulated with ppTG1 alone or with pcTG90 / DOPE 1:2 in the absence or the presence of 42 µg ppTG1. The final charge ratio of each formulation is indicated.

Figure 8: Transfection efficiency- Effect of JTS-1.

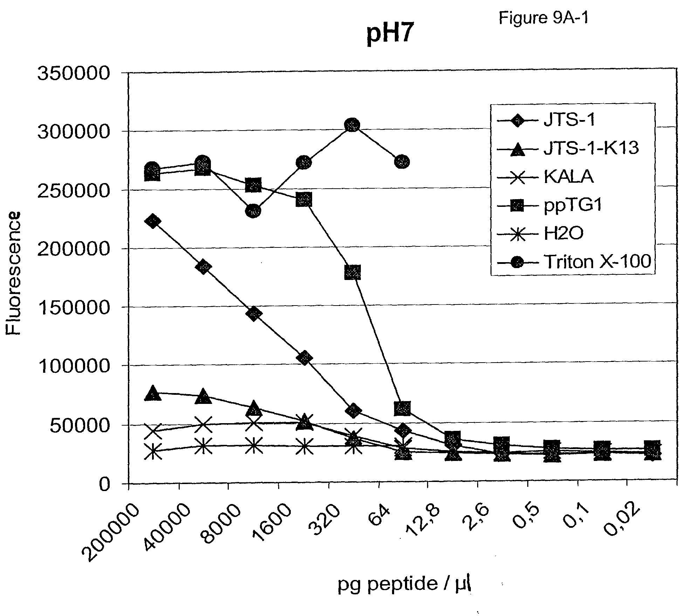

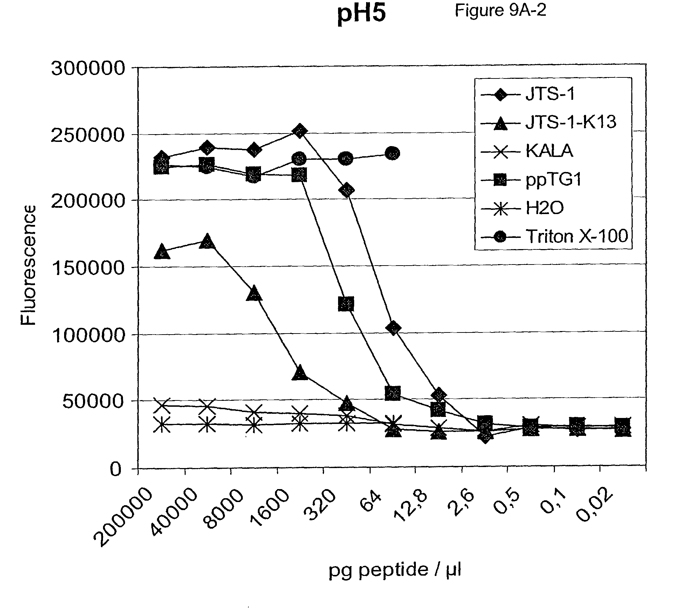

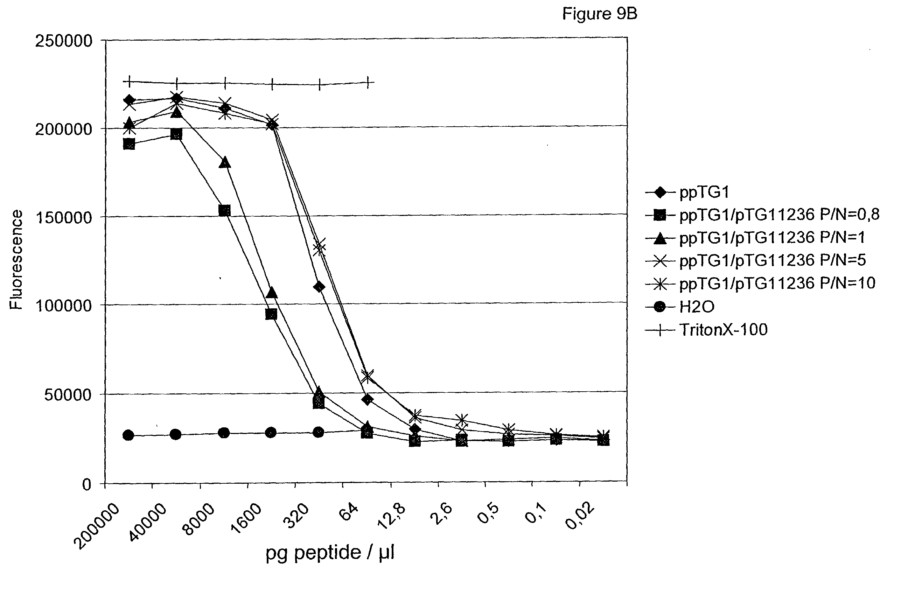

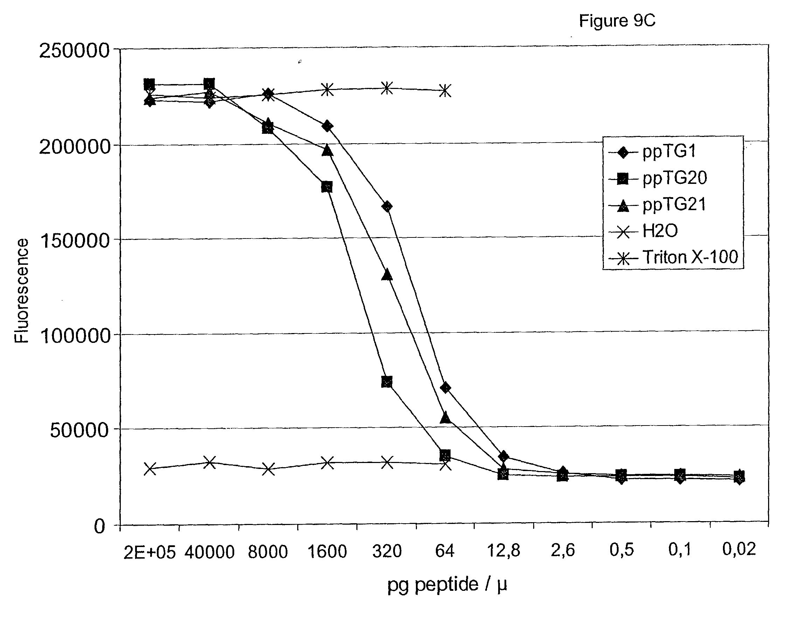

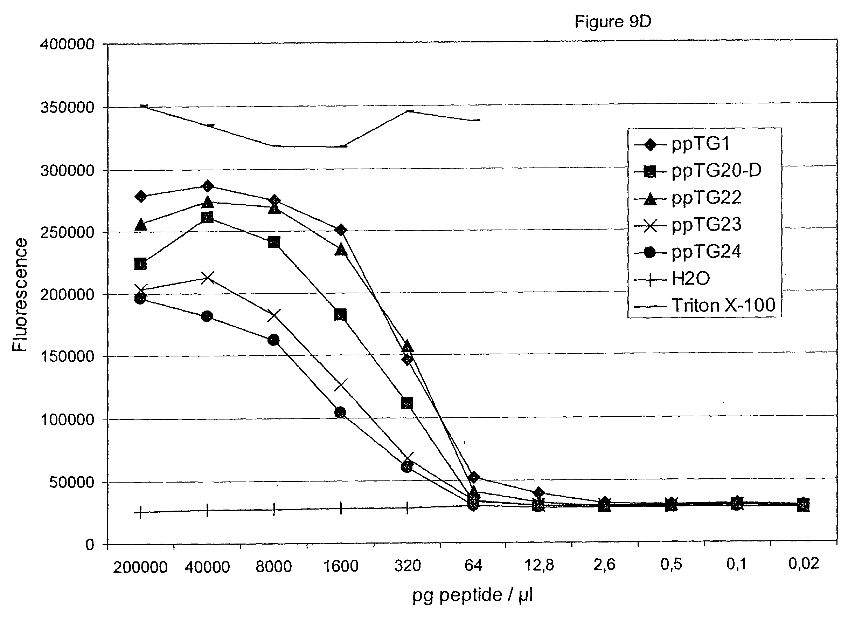

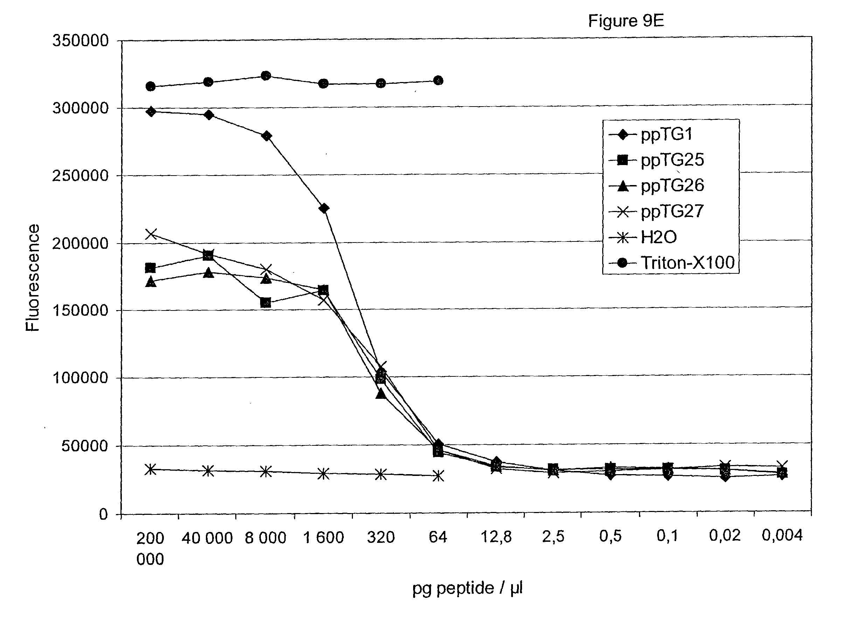

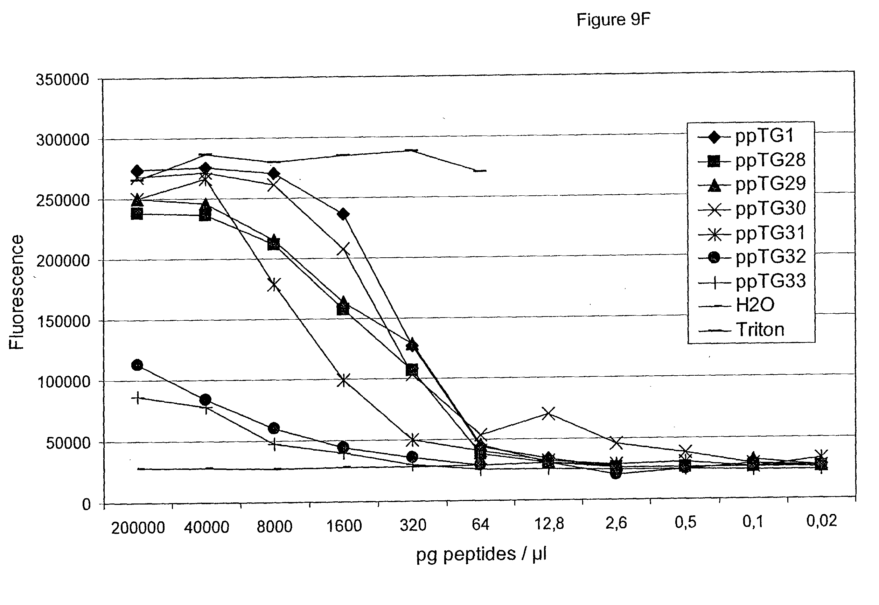

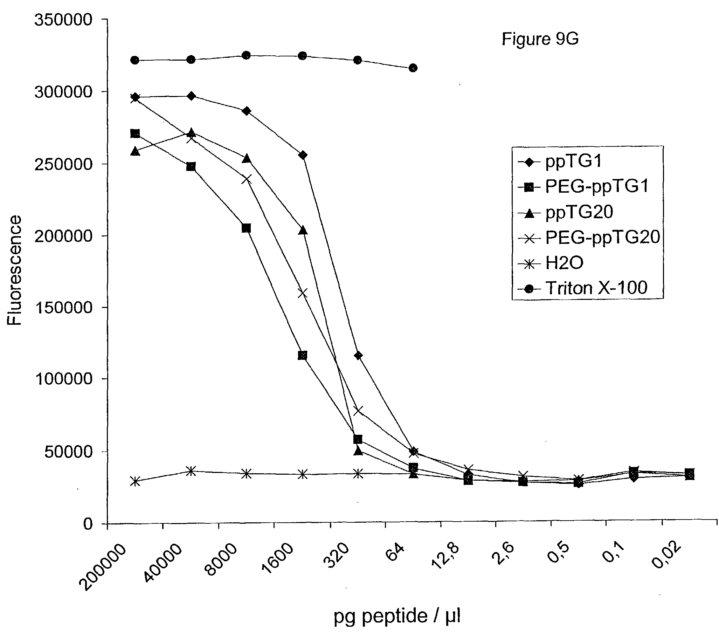

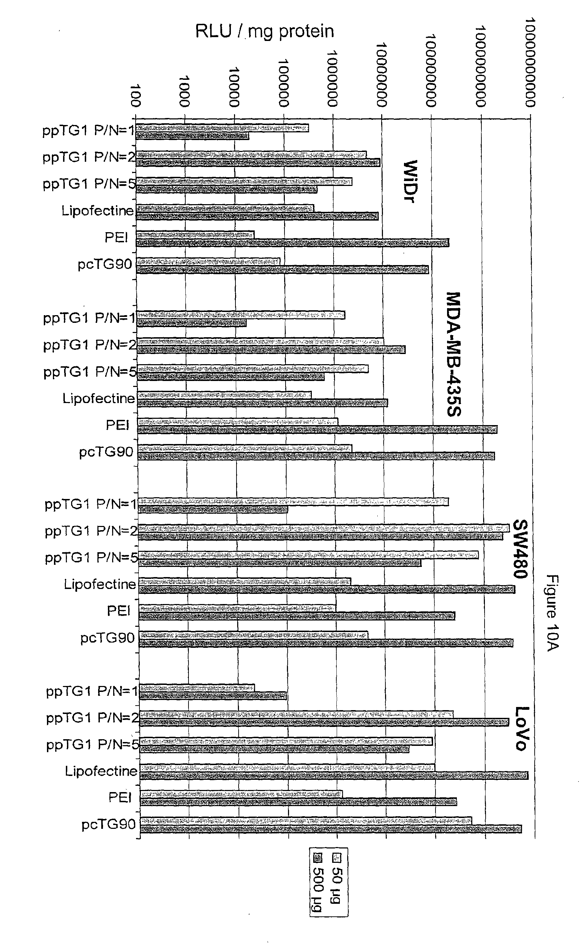

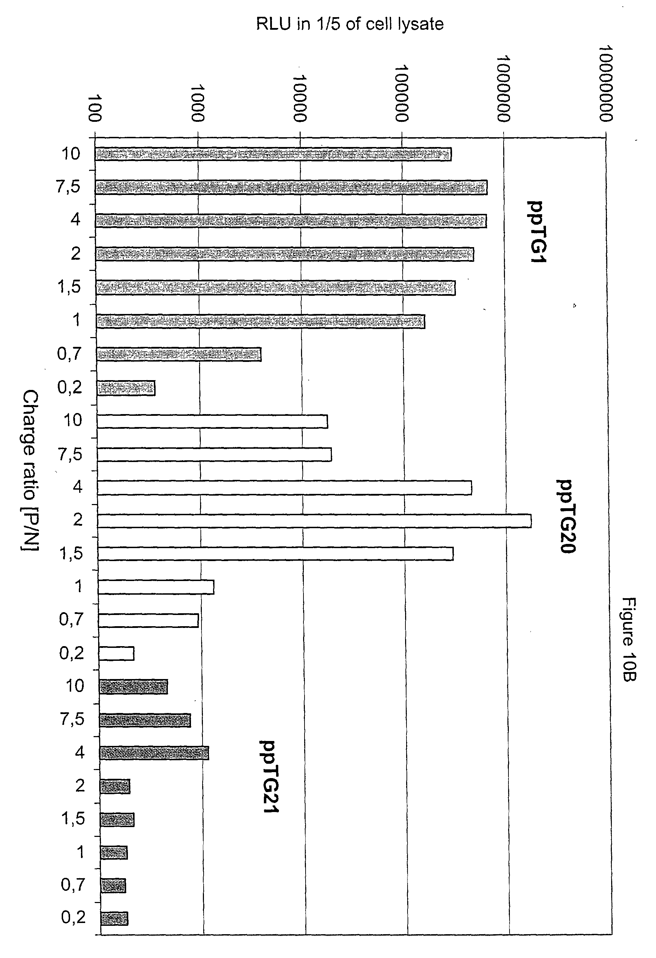

Figure 9: Liposome leakage assay. Increasing amounts of the indicated peptides were incubated with POPC/Cholesterol (3:2 mol/mol) liposomes for 30 min at RT. Emitted fluorescence was plotted against peptide concentration. A) Comparison of ppTG1, JTS-1-K13, KALA and JTS-1 at pH5 and pH7. B) Liposome leakage activity with complexes of ppTG1 and the plasmid pTG11236 at pH7. C) Comparison of ppTG1, ppTG20 and ppTG21 at pH7. D) Comparison of ppTG1, ppTG20-D, ppTG22, ppTG23 and ppTG24 at pH7. E) Comparison of ppTG1 with ppTG25, ppTG26 and ppTG27 at pH7. F) Comparison of ppTG1 with the series of peptides ppTG28 to ppTG33 at pH7. G) Comparison of ppTG1 and ppTG20 with PEG-ppTG1 and PEG-ppTG20 at pH7.

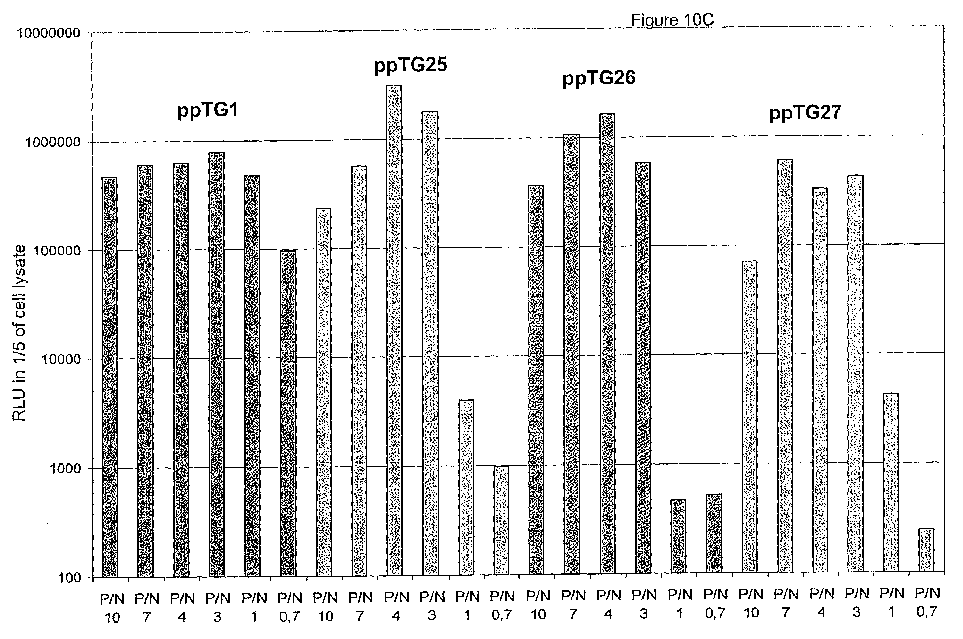

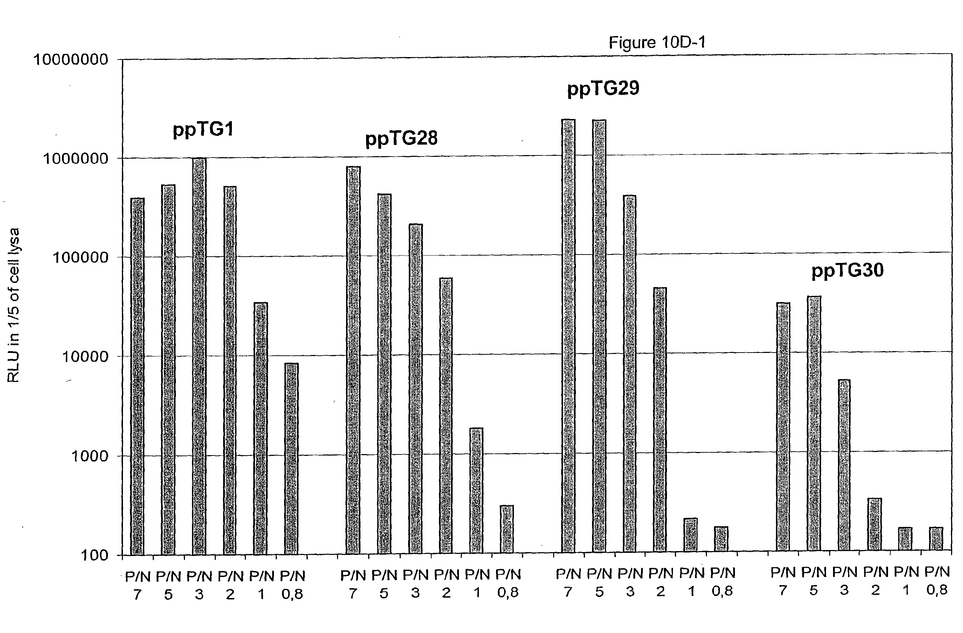

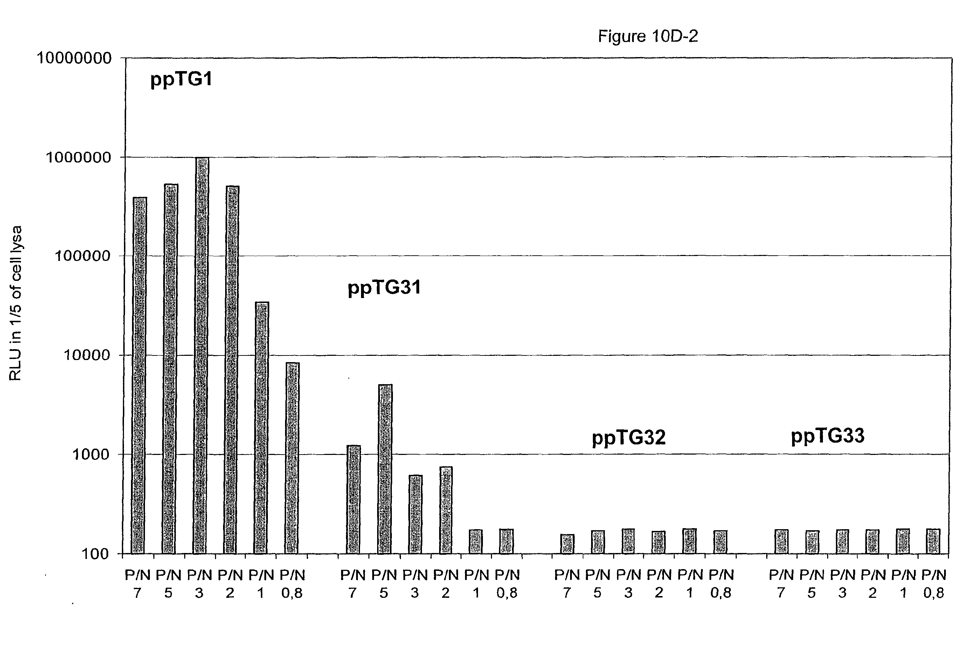

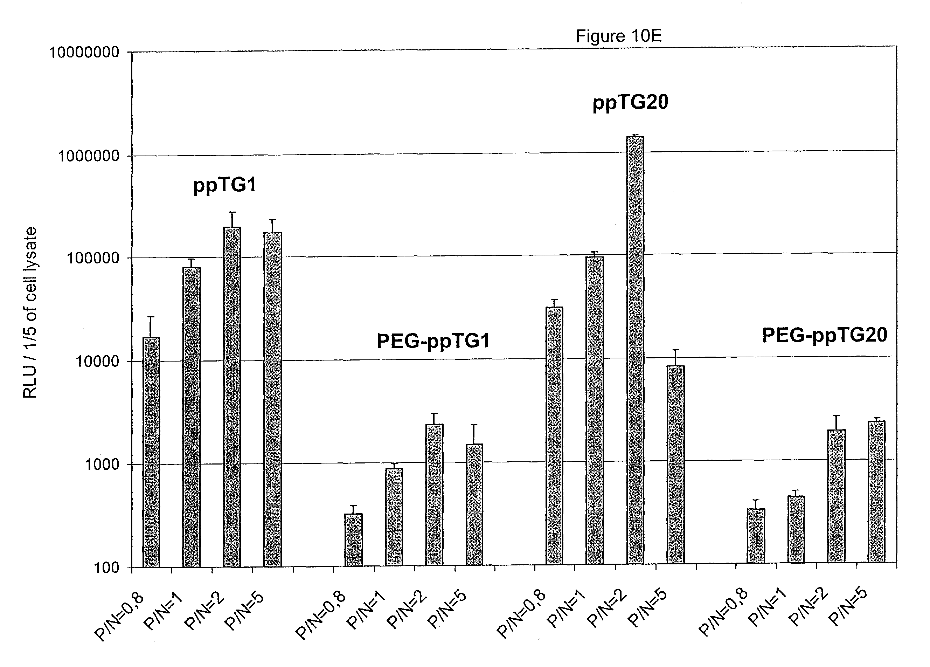

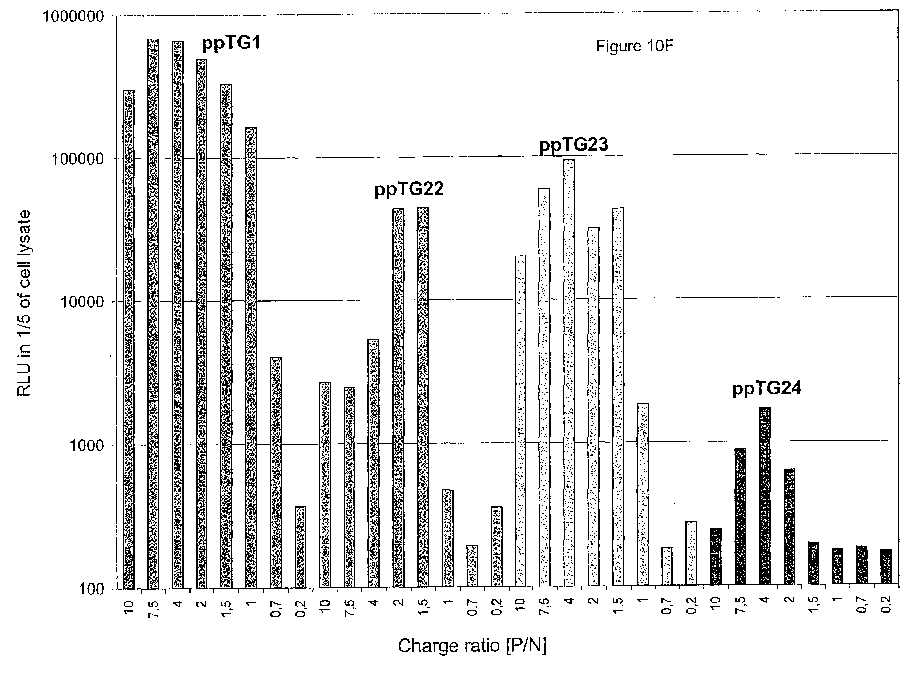

Figure 10: Transfection studies in vitro. A) The human tumor cell lines WiDr, MDA-MB-4355, SW480 and LoVo were transfected with 500ng or 50ng of the luciferase expression plasmid pTG11236 using ppTG1 at different charge ratios, PEI, Lipofectin and pcTG90/DOPE. The results of the luciferase assay at day 1 after transfection are indicated. B) to G) HeLa cells were transfected with 50 ng pTG11236 using the indicated peptides at increasing charge ratios [P/N]. B) ppTG1, ppTG20 and ppTG21. C) ppTG25, ppTG26 and ppTG27. D) ppTG1 and the series ppTG28 to ppTG33. E) ppTG1, PEG-ppTG1 and PEG-ppTG20. F) ppTG1, ppTG22, ppTG23 and ppTG24. G) ppTG1 and ppTG20-D.

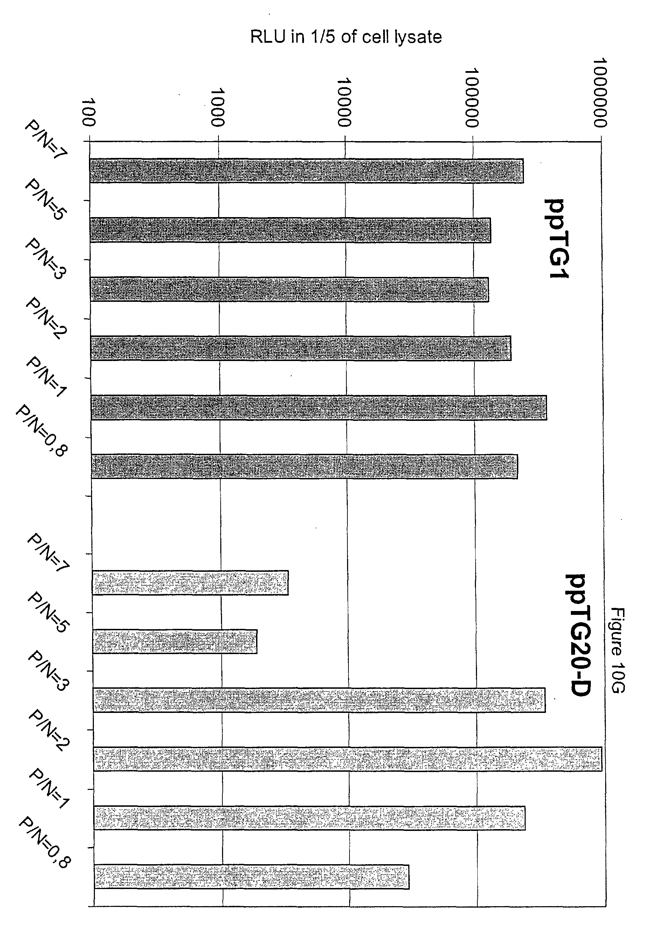

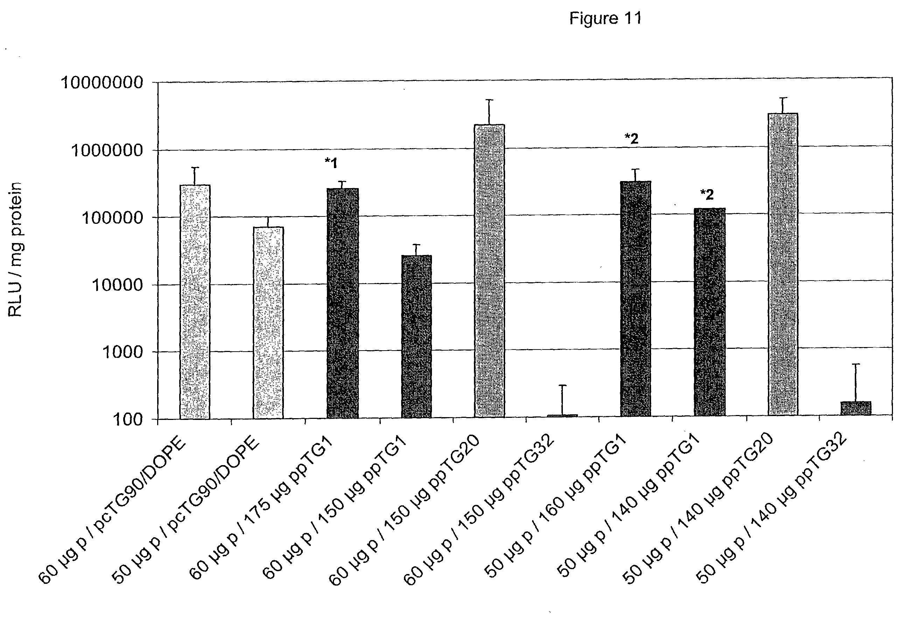

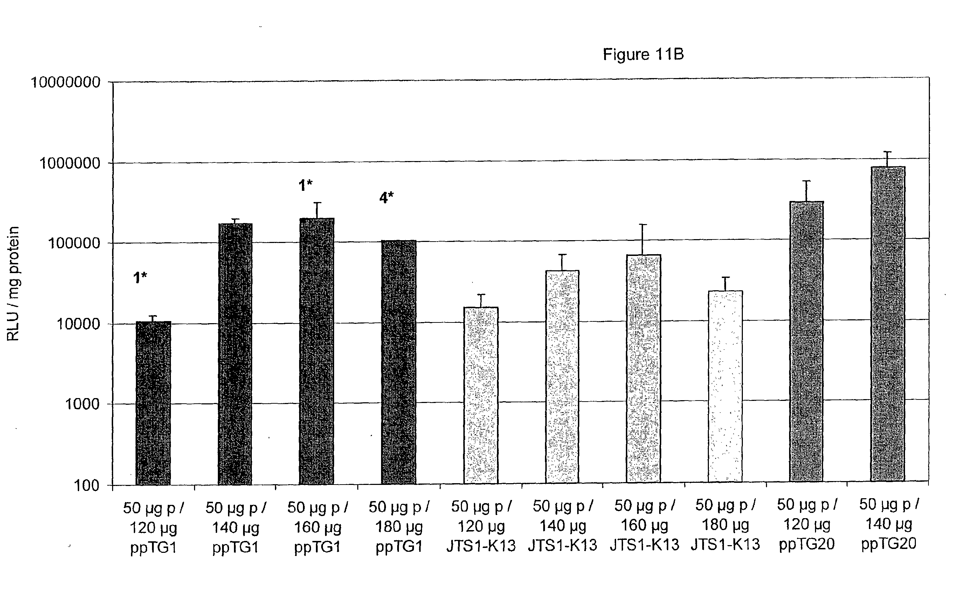

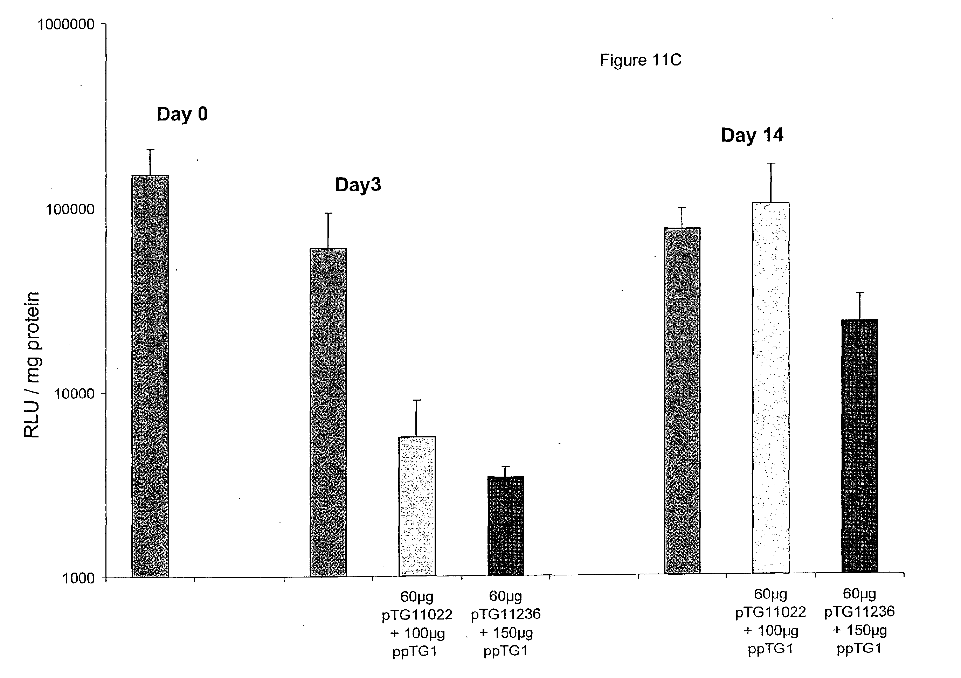

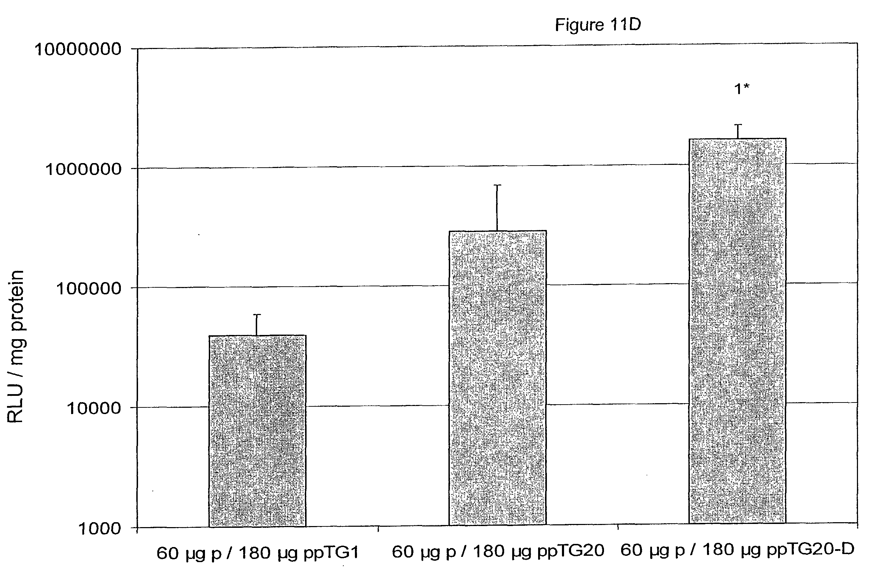

Figure 11: In vivo experiments. Numbers with an asterisk indicate the number of dead mice per group of five. A) Five B6SJL mice per group were intravenously injected with 60 µg or 50 µg of pTG11236 complexed with pcTG90/DOPE 1:2 [P/N] 10, or with the indicated amounts of ppTG1, ppTG20 and ppTG32. Mice were sacrificed day 1 after injection and luciferase activities in the lungs were analyzed. B) Five B6SJL mice per group were intravenously injected with 60 µg or 50 µg of pTG11236 complexed with ppTG1, JTS-1-K13 and ppTG20. Mice were sacrificed day 1 after injection and luciferase activities in the lungs were analyzed. C) All groups of five B6SJL mice were intravenously injected with 60 µg pTG11236 complexed with 150 µg ppTG1 at day 0, 3 and 14 after pre-injection of 60 µg pTG11236 or pTG11022 ("empty vector") complexed with ppTG1 at day 0. Mice were sacrificed the next day. Luciferase activities in the lung/mg protein are shown. D) Five B6SJL mice per group were intravenously injected with 60 µg pTG11236 complexed with 180 µg ppTG1, ppTG20 or ppTG20-D. Mice were sacrificed day 1 after injection and luciferase activities in the lungs are indicated.

### EXAMPLES :

In accordance with the present invention, a new low molecular weight cationic peptide has been synthesized, ppTG1 (SEQ ID NO:2). This peptide does not contain glutamic acid residues and is capable of binding and compacting DNA, and further of causing membrane disruption.

### Materials and Methods

**Cells :** HeLa cells (ATCC) and 293-EBNA cells (Invitrogen) were cultivated in DMEM medium supplemented with 10% fetal calf serum, 1% gentamycine, 1% glutamine and 3 g/l glucose, in an incubator at 37°C and 5% CO2.
WiDr (ATCC CCL-218), MDA-MB-435S (ATCC HTB-129), SW480 (ATCC CCL-228) and LoVo cell (CCL-229) were cultivated in the appropriate medium with 10% fetal calf serum, 1% gentamycine, 1% glutamine and 3 g/l glucose, in an incubator at 37°C and 5% CO₂.
**Plasmids** : The plasmid pTG11056 (13787 bp; Längle-Rouault et al., 1998, J. Virol., 72, 6181-6185) is employed which carries besides the EBV oriP sequences a luciferase gene under the control of the CMV promoter, intron 1 of the HMG gene and the SV40 polyA signal. Additionally, the plasmid pTG11236 (5738 bp) with a luciferase expression cassette comprising the CMV promoter, the short SV40 16S/19S intron and the SV40 polyA signal, is also used in the experiments. The plasmid pTG11022 (7998 bp) represents a plasmid with an "empty" CMV IE promoter-driven expression cassette, containing the intron1 of the HMG gene and the SV40 polyA signal.
**Polypeptides** : The chemical synthesis of the peptides ppTG1 (SEQ ID NO:2), JTS-1(SEQ ID NO:3), JTS-1-K13 (SEQ ID NO:4), ppTG20 (20-mer) (SEQ ID NO:6) and KALA (SEQ ID NO:5) was carried out by Neosystem (France).

ppTG1 (20-mer, MW 2297) (SEQ ID N0:2)
Gly-Leu-Phe-Lys-Ala-Leu-Leu-Lys-Leu-Leu-Lys-Ser-Leu-Trp-
Lys-Leu-Leu-Leu-Lys-Ala

JTS-1 (20-mer, MW 2301) (SEQ ID N0:3)
Gly-Leu-Phe-Glu-Ala-Leu-Leu-Glu-Leu-Leu-Glu-Ser-Leu-Trp-
Glu-Leu-Leu-Leu-Glu-Ala

JTS-1-K13 (40-mer, MW 4826) (SEQ ID N0:4)
Gly-Leu-Phe-Glu-Ala-Leu-Leu-Glu-Leu-Leu-Glu-Ser-Leu-Trp-
Glu-Leu-Leu-Leu-Glu-Ala-Cys_Cys-Tyr-Lys-Ala-Lys-Lys-Lys-
Lys-Lys-Lys-Lys-Lys-Trp-Lys-Lys-Lys-Lys-Gln-Ser

KALA (30-mer, MW 3131) (SEQ ID N0:5)
Trp-Glu-Ala-Lys-Leu-Ala-Lys-Ala-Leu-Ala-Lys-Ala-Leu-Ala-
Lys-His-Leu-Ala-Lys-Ala-Leu-Ala-Lys-Ala-Leu-Lys-Ala-Cys-
Glu-Ala

ppTG20 (20-mer) (SEQ ID N0:6)
Gly-Leu-Phe-Arg-Ala-Leu-Leu-Arg-Leu-Leu-Arg-Ser-Leu-Trp-
Arg-Leu-Leu-Leu-Arg-Ala

ppTG21 (20-mer) (SEQ ID NO :7)
Gly-Leu-Phe-His-Ala-Leu-Leu-His-Leu-Leu-His-Ser-Leu-Trp-
His-Leu-Leu-Leu-His-Ala

ppTG22 (21-mer) (SEQ ID NO :8)
Gly-Leu-Phe-Lys-Ala-Leu-Leu-Lys-Leu-Leu-Lys-Ser-Leu-Trp-
Lys-Leu-Leu-Leu-Lys-Ala-Cys

ppTG23 (21-mer) (SEQ ID NO :9)
Cys-Gly-Leu-Phe-Lys-Ala-Leu-Leu-Lys-Leu-Leu-Lys-Ser-Leu-
Trp-Lys-Leu-Leu-Leu-Lys-Ala

ppTG24 (22-mer) (SEQ ID NO :10)
Cys-Gly-Leu-Phe-Lys-Ala-Leu-Leu-Lys-Leu-Leu-Lys-Ser-Leu-
Trp-Lys-Leu-Leu-Leu-Lys-Ala-Cys

ppTG25 (20-mer) (SEQ ID NO :11) ppTG1-linker-SV40NLS
Gly-Leu-Phe-Lys-Ala-Leu-Leu-Lys-Leu-Leu-Lys-Ser-Leu-Trp-
Lys-Leu-Leu-Leu-Lys-Ala-Gly-Gly-Gly-Pro-Lys-Lys-Lys-Arg-
Lys-Val-Glu-Asp

ppTG26 (20-mer) (SEQ ID NO :12)ppTG1-linker-SV40NLSm
(mutated)
Gly-Leu-Phe-Lys-Ala-Leu-Leu-Lys-Leu-Leu-Lys-Ser-Leu-Trp-
Lys-Leu-Leu-Leu-Lys-Ala-Gly-Gly-Gly-Pro-Lys-Thr-Lys-Arg-
Lys-Val-Glu-Asp

ppTG27 (20-mer) (SEQ ID NO :13) ppTG1-linker-SV40NLSrev
(reversed)
Gly-Leu-Phe-Lys-Ala-Leu-Leu-Lys-Leu-Leu-Lys-Ser-Leu-Trp-
Lys-Leu-Leu-Leu-Lys-Ala-Gly-Gly-Gly-Asp-Glu-Val-Lys-Arg-
Lys-Lys-Lys-Pro

ppTG28 (20-mer) (SEQ ID NO :14)
Gly-Leu-Phe-Lys-Lys-Leu-Leu-Lys-Leu-Leu-Lys-Lys-Leu-Trp-
Lys-Leu-Leu-Leu-Lys-Ala

ppTG29 (20-mer) (SEQ ID NO :15)
Gly-Leu-Phe-Arg-Arg-Leu-Leu-Arg-Leu-Leu-Arg-Arg-Leu-Trp-
Arg-Leu-Leu-Leu-Arg-Ala

ppTG30 (20-mer) (SEQ ID NO :16)

Gly-Ile-Phe-Lys-Ala-Ile-Ile-Lys-Ile-Ile-Lys-Ser-Ile-Trp-
Lys-Ile-Ile-Ile-Lys-Ala

ppTG31 (20-mer) (SEQ ID NO :17)
Gly-Ile-Phe-Arg-Ala-Ile-Ile-Arg-Ile-Ile-Arg-Ser-Ile-Trp-
Arg-Ile-Ile-Ile-Arg-Ala

ppTG32 (20-mer) (SEQ ID NO :18)
Gly-Val-Phe-Lys-Ala-Val-Val-Lys-Val-Val-Lys-Ser-Val-Trp-
Lys-Val-Val-Val-Lys-Ala

ppTG33 (20-mer) (SEQ ID NO :19)
Gly-Val-Phe-Arg-Ala-Val-Val-Arg-Val-Val-Arg-Ser-Val-Trp-
Arg-Val-Val-Val-Arg-Ala

ppTG20-D-configuration (20-mer) (SEQ ID NO :20)
Gly-Leu-Phe-Arg-Ala-Leu-Leu-Arg-Leu-Leu-Arg-Ser-Leu-Trp-
Arg-Leu-Leu-Leu-Arg-Ala

The peptides were received as lyophilized powder at a purity of 80 - 97% with acetate as counter-ion in case of the cationic peptides. The peptide JTS-1-K13 was synthesized in two steps. First: synthesis of JTS-1-Cystein and Cystein-K13, then formation of disulfide bridge. All peptides are diluted in milliQ water to a final concentration of at least 1µg/µl.

When necessary, the N-termini of the peptides ppTG1 and ppTG20 were covalently linked to polyethyleneglycol (PEG) MW 2000 resulting in PEG-ppTG1 and PEG-ppTG20. These products were not HPLC-purified. All peptides were dissolved in milliQ water if not indicated differently at a concentration between 3.3 µg/µl and 0.5 µg/µl.

### Lipid and colipid

The lipid pcTG90 is as disclosed in EP 901463 the content of which is incorporated herein in its entirety (see also the formula provided above).

### Gel retardation assay

3*µ*g of the plasmid pTG11236 were incubated with increasing amounts of the respective peptide in a final volume of 30*µ*l with 0.9 % NaCl. After 20 min of incubation at room temperature, 6*µ*l 5x loading buffer (glycerol and bromphenol blue in TAE buffer) were added and 10 *µ*l of these solutions were analyzed on a 1% agarose gel in the presence of ethidium bromide.

### Liposome leakage assay

1-Palmitoyl-2-Oleoyl-phosphatidylcholine (POPC) liposomes were prepared by the repeated freeze-thaw method followed by extrusion (Olson et al., 1979, Biochim. Biophys. Acta 557, 19-23). Ten *µ*-moles of POPC in chloroform were placed in a glass tube and solvent was evaporated under reduced pressure using a Labconco Rapidvap vortex evaporator (Uniequip, Germany). The resulting lipid film was hydrated with 0.5 ml of an aqueous solution of calcein (100mM calcein, 3.75 eq. NaOH, 50mM NaCl). The lipid suspension was sonicated until the solution became clear using a sonic water bath (Bransonic 221, Branson Ultrasonics Corp., USA). Five cycles of freeze-thaw were performed before liposomes were extruded by 4 passages through 200nm pore diameter polycarbonate membranes (Nuclepore, Costar, MA, USA). Free calcein was removed from calcein encapsulated into liposomes by gel exclusion chromatography using a Sephadex G-50 column (3cm x 14 cm) and a 200mM NaCl, 25mM HEPES, pH 7.3 solution as the elution buffer. The final lipid concentration was determined by a phosphorus assay (Bartlett, 1959, J. Biol. Chem. 234, 466-469). Liposomes' diameter was determined by dynamic laser light scattering using a Coulter N4 Plus submicron particle sizer (Coultronics France S.A, France). Measurements were performed within a 3nm -10 000nm size window with a fixed 90° scattered light angle.

The liposome leakage assay was carried out as described in Planck et al. (1994, J. Biol. Chem. 269, 12918-12924). The liposome stock solution was diluted to a lipid concentration of 45 *µ*M in 1.8 x assay buffer (360mM NaCl, 36 mM sodium citrate, pH 5 and pH 7). A 1:5 serial dilution of the tested peptide was carried out in a 96-well microtiter plate by transferring 20 *µ*l of the peptide solution from one well to the next well and diluting with 80 *µ*l H₂O. 100 µl of the liposome solution was added in each well (final lipid concentration: 25 *µ*M) and , after 30 min at room temperature, was assayed for fluorescence at 535nm (excitation: 485nm) on a microtiter plate fluorescence spectrometer (WALLAC, 1420 multilabel counter Victor). These conditions are appropriate to analyze fluorescein and as close as possible to the appropriate excitation / emission profile of calcein (495nm /515nm). The value for 100 % leakage was obtained by addition of 1 *µ*l of a 10% Triton X-100 solution. Leakage activity was plotted against peptide concentration.

### Transfection assays with fusogenic peptides

Cells were plated on 24 well plates at a density of 4 x 10⁴ (293-EBNA ) or 7 x10⁴ (HeLa, Renca) cells / well in DMEM supplemented with 10% FCS. The next day, the medium was replaced by 200 *µ*l serum-free DMEM and plasmid (pTG11056 or pTG11236) / peptide complexes or plasmid / peptide /lipid complexes were prepared in 30*µ*l 0.9 % NaCl or 5% glucose. After 20 min at room temperature these complexes were added to the cells which were then incubated for 2-3 h at 37°C and 5% CO₂ before 1 ml serum-containing DMEM was added. Approximately 20 hours later, cells were lysed by addition of 100 *µ*l 1x Promega lysis buffer, 20 *µ*l of the lysates were analysed for luciferase activity. Proteins were quantified with the bicinchonic acid (BCA) colorimetric method (Smith, Anal. Biochem. 150 (1985), 76-85).

### In vivo experiments

Indicated amounts of the luciferase expression plasmid pTG11236 were mixed with ppTG1 and /or pcTG90/DOPE. The resulting formulations were injected intramuscularly (30*µ*l), intravenously (250*µ*l) into mice or intratumorally (Renca tumors, 100*µ*l). Muscles, tumors or organs were recovered at the indicated points of time, macerated and tested for luciferase activity with the luciferase assay kit from Promega.

### Cell culture

Transfection in the presence of Bafilomycin A: 6x10⁴ HeLa cells were plated on 24 well plates. Cells were treated with 175 nM Bafilomycin A 30 min before and throughout the transfection (1h incubation of cells with transfection mix in the absence of serum).

### Example 1: DNA-binding activity

The ability of ppTG1 peptide to complex DNA was analyzed by gel retardation assays. 3 *µ*g pTG11236 were mixed with increasing amounts of ppTG1 (0.01*µ*g to 27 *µ*g) adding 0.9% NaCl to a final volume of 30 *µ*l. After 20 minutes incubation at room temperature, loading buffer was added and 10 µl of the resulting solution were analyzed on a 1% agarose gel. Similar studies were carried out with JTS-1, JTS-1-K13 and KALA peptides.

The results have shown that it is possible to obtain the retardation of plasmid pTG11236 when complexed with ppTG1 at a plasmid/peptide ratio of 3 *µ*g plasmid DNA (0,8 pmol) to 4 *µ*g peptide (1,4 nmol). 50 % of the DNA is complexed when adding 1 *µ*g peptide (0,35 nmol). Retardation of plasmid DNA in the presence of the anionic JTS-1 was not observed (3*µ*g pTG11236 and 0.1*µ*g to 10*µ*g JTS-1), while 3*µ*g (0,6 nmol) JTS-1-K13 led to >95% retardation of 3*µ*g pTG11236. These results show that the peptide ppTG1 of the invention is able to form complexes with DNA plasmid.

### Example 2: Liposome leakage activity

The peptide ppTG1 was tested for its capacity to cause membrane disruption in a liposome leakage assay with POPC liposomes containing the fluorescent product calcein. The liposome leakage assay was carried out as described in Planck *et al.* (1994- *supra).* Release of calcein by Triton X-100 treatment was taken as positive control reaction, incubation with water served as negative control. The membranolytic activity of peptide ppTG1 (20 *µ*g as starting point) was compared with those of equal quantities (20 *µ*g) of JTS-1, JTS-1-K13 and KALA.

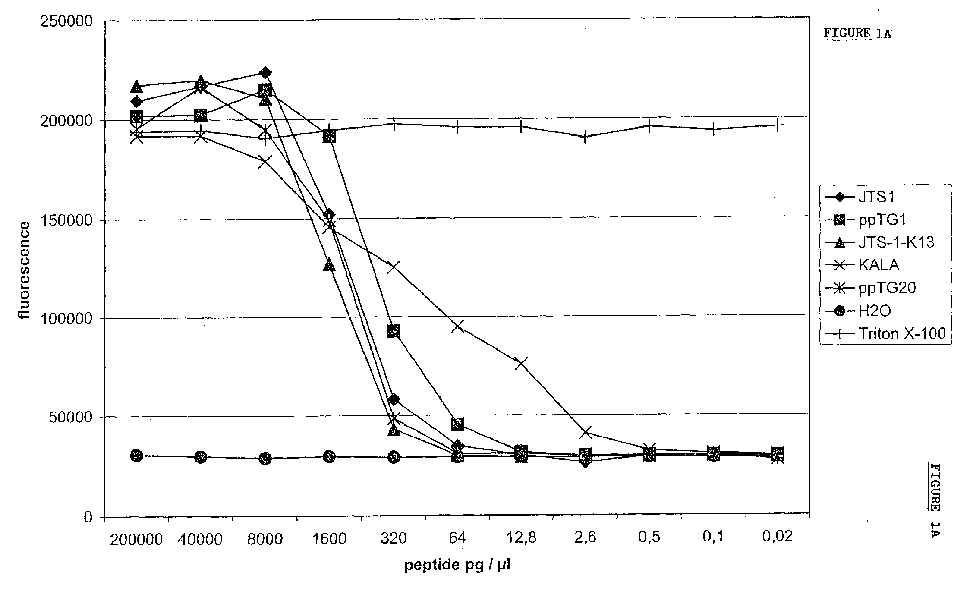

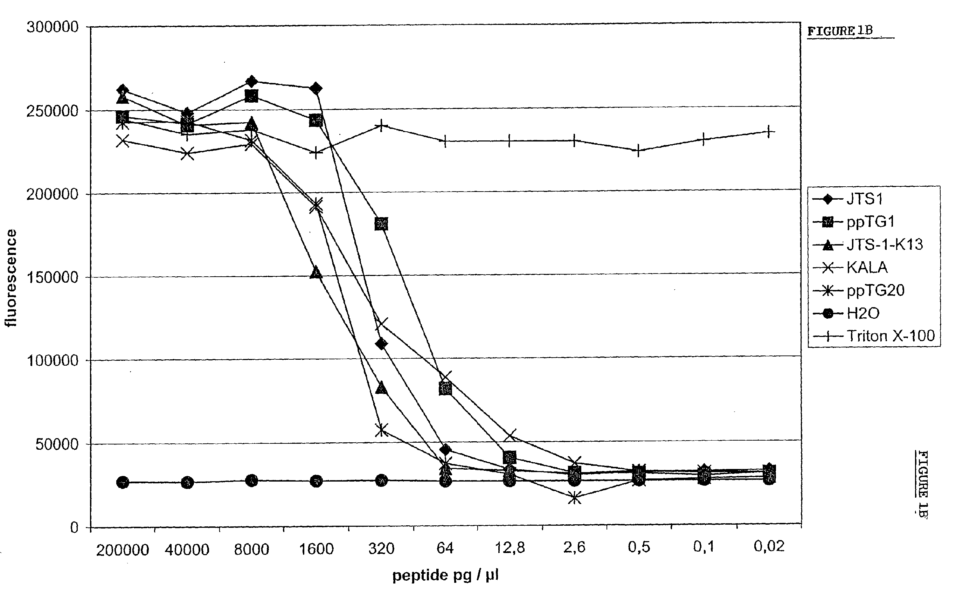

The results are presented in Figures 1A/1B.

Figures 1A/1B show that ppTG1 is capable of causing membrane disruption both at pH5 (Figure 1A)and pH7 (Figure 1B), at least as efficiently as JTS-1 and JTS-1-K13 did.

### Example 3: Transfection efficiency in vitro - comparison of ppTG1, Lipofectin and PEI.

Complexes of ppTG1 and plasmid DNA were analyzed for their ability to transfect 293-EBNA1 and Hela cells *in vitro*.

4x10⁴ 293-EBNA cells, plated on 24 well plates the day before, were transfected with 0.5 *µ*g, 0.1*µ*g, 0.05*µ*g and 0.01 *µ*g of the plasmid pTG11056. The plasmid was either mixed with completely or uncompletely DNA-complexing amounts of ppTG1 in 30 *µ*l 5% glucose and after 20 min at room temperature the mixture was added to the cells which were incubated in 200 *µ*l serum-free medium. Further, pTG11056 was formulated with the established transfection reagents Lipofectin and PEI. Lipofectin (Gibco BRL) and PEI were used as recommended by the manufacturer. Briefly, Lipofectin was added to plasmid DNA in a fourfold weight excess in 200 *µ*l serum free medium, which was then (after 20 min at room temperature) added to the cells. PEI was diluted to a 10mM solution, e.g. 75*µ*l were added to 0.5*µ*g DNA in 30*µ*l 5% glucose, after 30 min at room temperature transfer on cells incubated in 200*µ*l serum-free medium. Three hours later, 1 ml of serum-containing DMEM was added to the cells. After twenty hours, cells were washed and luciferase activity was determined in 1/5 of the lysed cells.

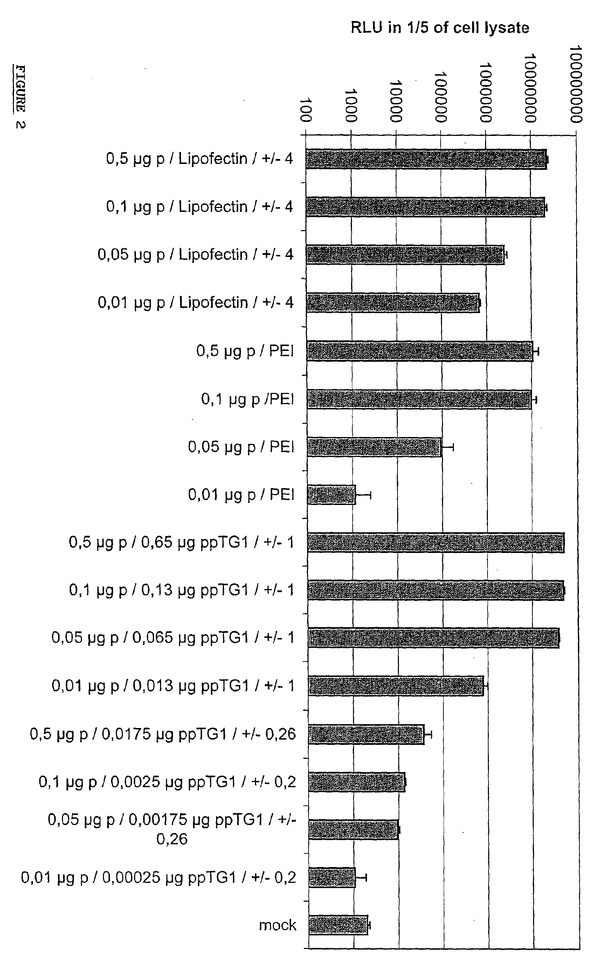

The luciferase activities are shown in Figure 2.

Figure 2 shows that transfection of 293-EBNA1 cells with 0.5 *µ*g pTG11056 complexed with 0.65 *µ*g ppTG1 (charge ratio around 1) led to luciferase activities higher than those observed with complexes formed with Lipofectin or PEI. Transfection with 0.05 µg pTG11056 mixed with 0.065 *µ*g ppTG1 (same charge ratio) still showed high transfection efficiency, while PEI and even Lipofectin formulations were at least 10-times less efficient.

This experiment shows that transfection with the complex of the invention comprising the peptide ppTG1 is at least as efficient, in most of the measurements even pronouncedly more efficient as with the complex of the prior art comprising Lipofectin or PEI especially at lower concentrations. ppTG1 alone is sufficient to efficiently transfect the cells with plasmid DNA.

In another experiment, 7x10⁴ HeLa cells, seeded on 24-well plates the day before transfection, were transfected with 0.5 *µ*g or 0.05 *µ*g pTG11236, complexed with Lipofectin or ppTG1. The plasmid / peptide complexes were prepared in 30*µ*l of 5% glucose or 0.9% NaCl and added to the cells (200 *µ*l serum-free medium) after 20 min at room temperature. Serum-containing medium was added after 3 h, the cells were harvested the next day.

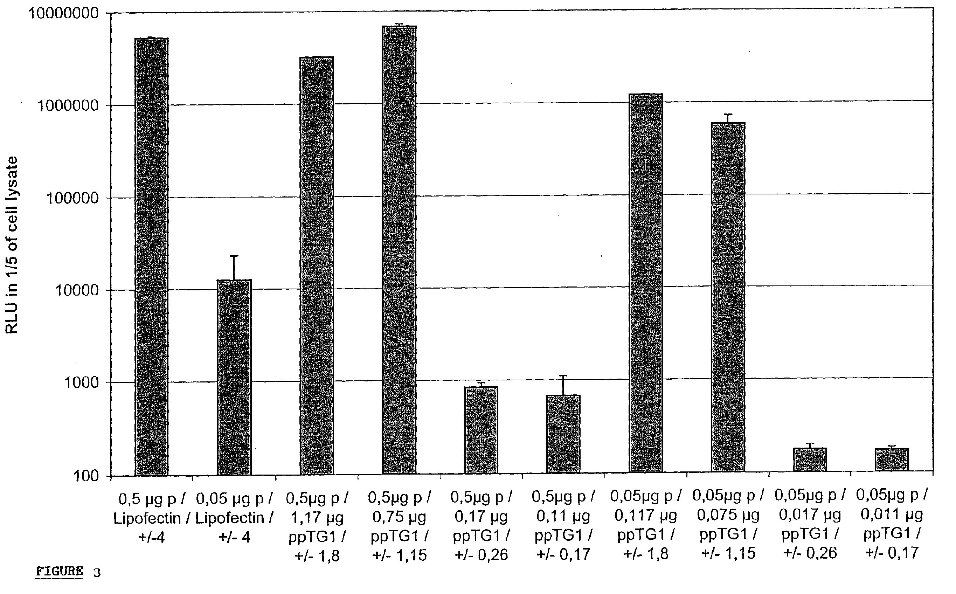

The luciferase activities in the total protein lysate are presented in Figure 3.

Figure 3 shows that the formulation of 0.5 *µ*g or 0.05 *µ*g of pTG11236 with 1.17*µ* g or 0.117*µ*g of ppTG1 (totally DNA-complexing amount of peptide; charge ratio 1.8) resulted in comparable luciferase activities as observed for DNA formulated with Lipofectin. Comparing transfection efficiencies of 0.05 *µ*g pTG11236, it appeared that plasmid DNA mixed with ppTG1 in 0.9% NaCl resulted in higher luciferase activities than observed for Lipofectin formulations.

Figure 2 and figure 3 demonstrate, that the peptide ppTG1 alone is sufficient to efficiently transfer plasmid DNA into cells. At low DNA dose this efficiency is superior to established transfection reagents such as for example Lipofectin or PEI reagents.

### Example 4: Transfection efficiency : comparison of ppTG1 and JTS-1-K13 in Hela cells

A comparison of the effect on transfection efficiency of ppTG1 and JTS-1-K13 was carried out in HeLa cells. Complexes of 0.5 *µ*g or 0.05 *µ*g pTG11236 were formed with increasing amounts the respective peptide.

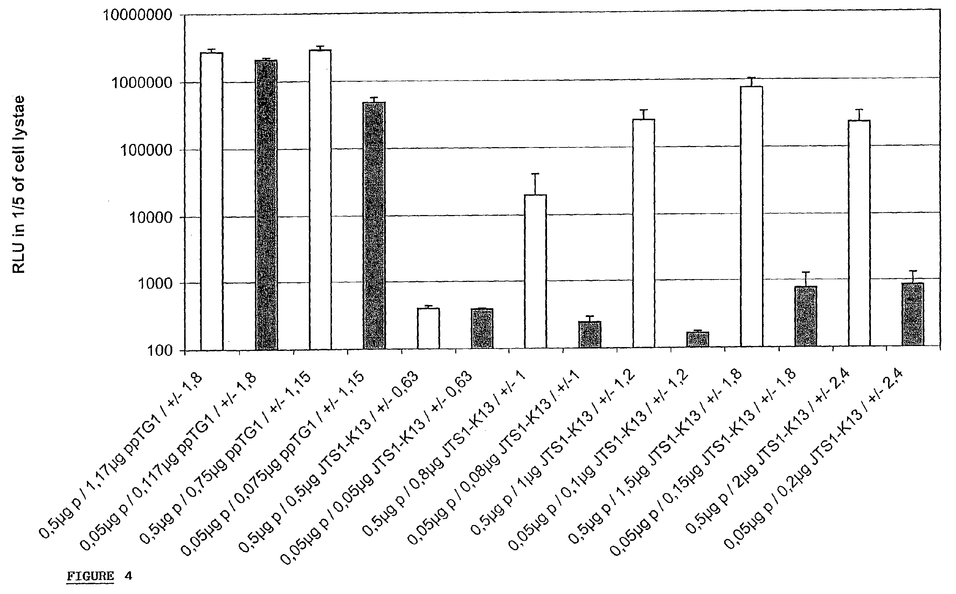

The results of the luciferase analysis are shown in Figure 4.

Figure 4 shows that while a high level of luciferase expression was obtained with 0.5*µ*g pTG11236 complexed with 1.5*µ*g JTS-1-K13, transfection with 0.05*µ*g plasmid DNA complexed with JTS-1-K13 remains low in contrast to transfection with ppTG1. These results indicate that ppTG1 is a better transfection reagent than JTS-1-K13.

### Example 5: Transfection efficiency in vitro - comparison of ppTG1 and KALA in HeLa and CHO cells

A comparison of ppTG1 and KALA was carried out in HeLa and CHO cells. 5x10⁴ cells were seeded on 24 well plates. The next day, cells were transfected with 500ng and 50ng of pTG11236, complexed with ppTG1 or KALA in 30*µ*l 0.9% NaCl. The charge ratio +/- varied from 1, 2, 3, 4, 7 to 10. Cells were harvested 20h after transfection and lysis was obtained in 100 *µ*l Promega lysis buffer. Luciferase activity and total protein concentrations in 20 *µ*l were determined.

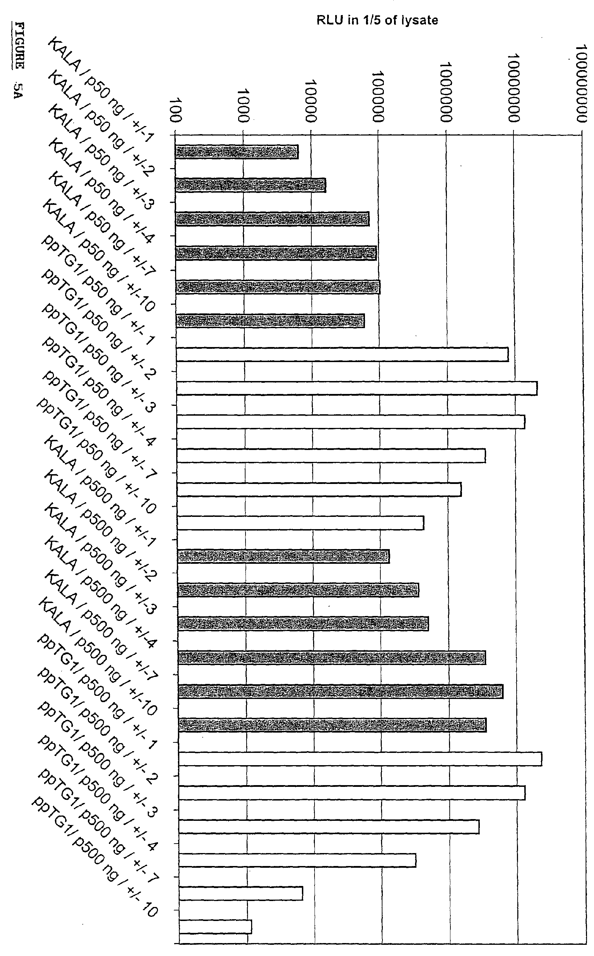

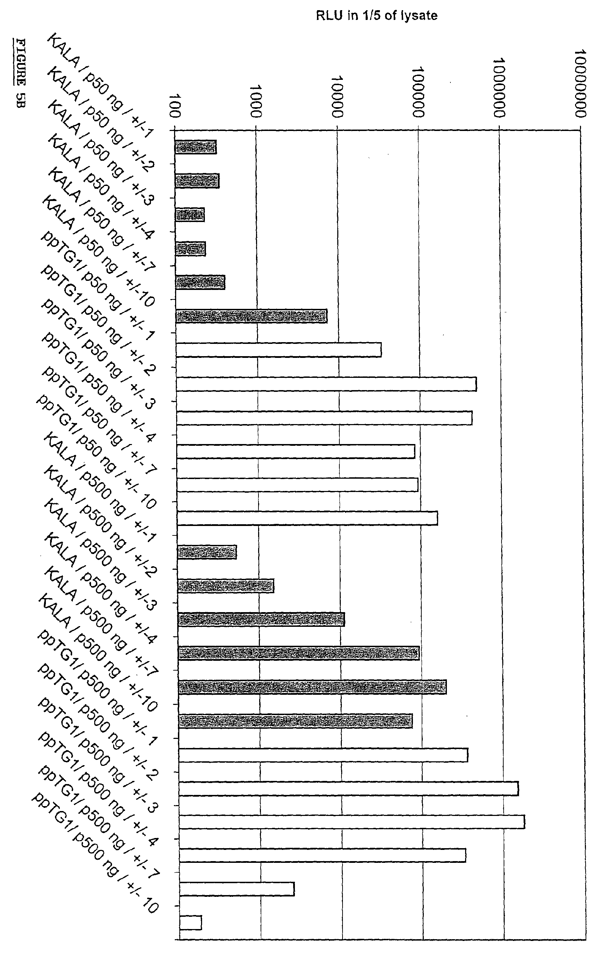

The results are presented in Figures 5 a/b.

Figures 5 a/b show that transfection with complexes comprising ppTG1 was more efficient than with those formed with the peptide KALA. For the peptide ppTG1, the best charge ratio condition in HeLa cells was either 1 or 2. KALA showed optimal gene transfer at a charge ratio of 7 which was 200-fold (50 ng pTG11236) to 4-fold (500ng) lower than for ppTG1. In the best case, the complex comprising ppTG1 was 3000-fold more efficient than the complex comprising KALA.

In CHO cells, the optimum of transfection was observed at charge ratios of 2 and 3, respectively, for complexes comprising ppTG1 and at charge ratios of 10 and 7, respectively, for complexes comprising KALA, with ppTG1 being more efficient in gene transfer (68-fold at 50ng pTG11236 and 9-fold at 500ng pTG11236). In the best case, the complex comprising ppTG1 was 2500-fold more efficient than the complex comprising KALA.

### Example 6: Transfection efficiency in vitro of complexes comprising peptide ppTG1 and pcTG90 / DOPE

A mixture of pcTG90 and DOPE (1:1) was prepared in 350*µ*l of chloroform. The solution was evaporated using the vortex evaporator. The lipid film obtained was resuspended in 1ml of 5% glucose to a concentration of about 0.5mg/ml. ppTG1 was dissolved to 3mg/ml in water and added to the lipide suspension. The mixture was then added to the DNA (pTG11236) diluted in 5% glucose, and vortexed.

HeLa cells were seeded at a density of 6 x 10⁴ cells on 24 well plates. The next day, cells were incubated with 200*µ*l serum-free medium, 30*µ*l of plasmid (50ng) /peptide / lipid mixtures were added and incubated for 3h at 37°C in 5% CO₂. 1ml of DMEM+10% FCS was then added. The next day, the medium was removed and cells were washed with 500*µ*l of PBS and subsequently treated with 100*µ*l of Promega lysis buffer. Plates were stored at -80°C until luciferase activity was measured of 20*µ*l of the cell lysate. The protein assay was performed using the Pierce BCA kit.

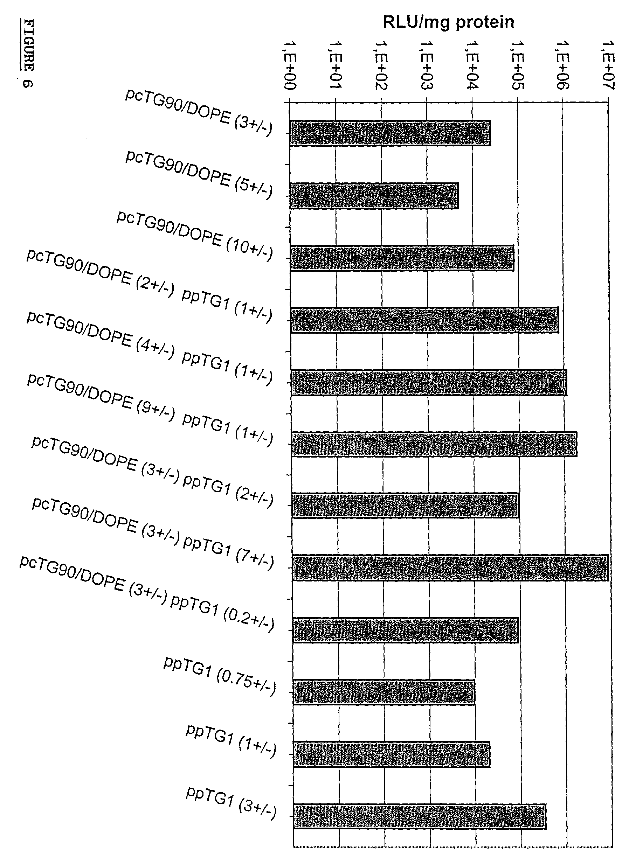

Figure 6 presents the results of this experiment.

Figure 6 shows that at 50ng of plasmid and a final charge ratio of 3, 5 and 10, the addition of small amounts of ppTG1 (contributing 1/3, 1/5 and 1/10, respectively, of the positive charge of the complex) improved by at least 1 log the transfection efficiency of pcTG90/DOPE. This improvement was even better (2 log) when the quantity of ppTG1 was increased (contributing 2/3 and 7/10, respectively, of the positive charge of the complex) at a final charge ratio of 5 and 10. At a final charge ratio of 3, ppTG1 alone gave better results than pcTG90/DOPE alone.

### Example 7: In vivo experiments

60 *µ*g or 30*µ*g of the plasmid pTG11236 were mixed with peptide ppTG1 and/or with a pcTG90 / DOPE 1:2 mixture in 250*µ*l 5% glucose. After 20 min incubation at room temperature, the complexes were intravenously injected into B6SJL mice. Mice were sacrificed at day 1. The lungs were recovered, total protein was extracted and luciferase activity was analysed.

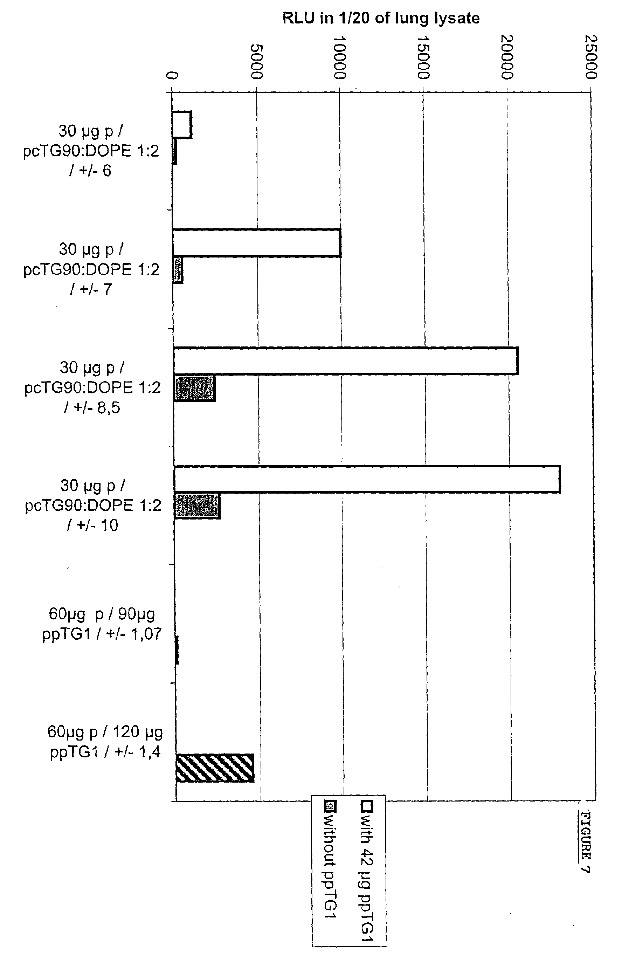

The results are shown in Figure 7.

Figure 7 shows that gene expression into the lung can be achieved with complexes comprising pTG11236 and ppTG1 (in 5 out of 5 mice). The presence of ppTG1 in complexes further comprising cationic lipids improved gene expression by a factor of 10.

### Example 8: Transfection efficiency- Effect of JTS-1

JTS1 was dissolved to 1mg/ml in 1mM NaOH and mixed with DNA diluted in 5% glucose. ppTG1 was then added to this solution. The pcTG90/DOPE ppTG1 mixtures were prepared as described in Example 6. Transfection assays were performed on HeLa cells with 50ng of plasmid as described in Example 6.

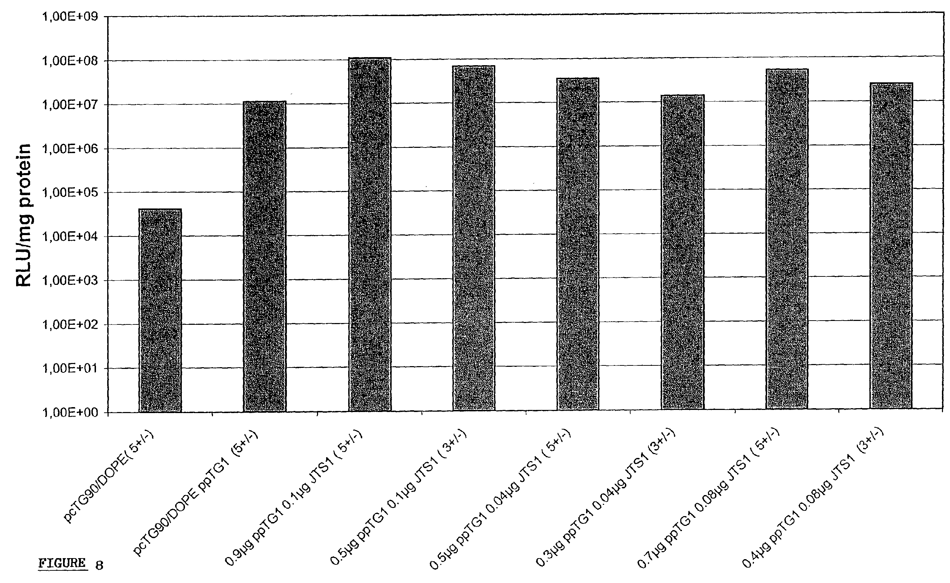

The results are shown on Figure 8.

Figure 8 shows that pTG11236 complexed with 0.9*µ* g of ppTG1 and 0.1*µ*g of JTS1 (final charge ratio 5) increased luciferase activity by a factor of about 10 in comparison with the best formulation of ppTG1/pcTG90/DOPE (final charge ratio 5, ppTG1 contributing a charge ratio of 2 (+/-), and by a factor of about 1000 in comparison with pcTG90/DOPE alone at a final charge ratio of 5.

ppTG1/ plasmid DNA complexes with a final charge ratio of 1-2 mediate efficient transfection of a variety of cell lines. The efficiency is comparable to, or superior to, transfection levels observed with complexes comprising Lipofectin, PEI, or multicomponent peptide complexes according to Gottschalk et al., 1996.

### Examples Summary :

### In vitro

The peptide ppTG1, alone or complexed with plasmid DNA, destabilizes efficiently POPC/Chol (3:2, mol:mol) liposomes, while KALA shows no activity on this type of liposomes. The analysis of gene transfer efficiencies was expanded to the human tumor cell lines WiDr, MDA-MB-4355, SW480 and LoVo. All these cell lines were successfully transfected with ppTG1/DNA complexes. Transfection efficiency with ppTG1/plasmid complexes was not diminished in the presence of Bafilomycin A. This indicates that gene transfer does not depend on the acidification of the endosomes.

Various derivatives of ppTG1 were designed. All these peptides (see Materials and Methods) were capable to retard the migration of plasmid DNA in an agarose gel at pH8, except ppTG21 (Lys⇒His) which binds plasmid DNA at pH5. ppTG20 (Lys⇒Arg) and ppTG21 were comparable to ppTG1 with respect to their liposome leakage activity on POPC/Chol liposomes. Transfection efficiency with ppTG20 was comparable to that of ppTG1, while transfection efficiency with ppTG21 was strongly reduced. The peptides ppTG28 and ppTG29, with two additional basic amino acid residues were comparable to ppTG1 and ppTG20 in liposome leakage and gene transfer assays. Replacement of Leu by Ile or Val diminished liposome leakage activity (ppTG32 and ppTG33), and diminished gene transfer efficiency in vitro (ppTG30, ppTG31, ppTG32 and ppTG33). ppTG1 derivatives with wild type or mutated basic nuclear localisation signal (SV40 large T antigen) at the C-terminus retained liposome leakage and gene transfer efficiency. The addition of Cys residues N- and/or C-terminal of ppTG1 did not abolish liposome leakage activity, gene transfer efficiencies were reduced. ppTG1 and ppTG20 derivatives with polyethylenglycol (PEG)2000 covalently linked to the N-terminus of the peptides retained the liposome leakage activity, gene transfer efficiencies, however, were strongly reduced. Further tested was a ppTG20 derivative with all amino acids in D-configuration (ppTG20-D). Liposome leakage and gene transfer activities with this peptide were retained.

### In vivo:

Complexes of plasmid DNA with the peptides ppTG20 and ppTG20-D, intravenously injected into mice, led to gene transfer efficiencies in the lung even higher than with ppTG1. Gene transfer, however, was low with JTS1-K13, and was even undetectable with KALA. The use of ppTG32 drastically reduced gene transfer efficiency, indicating that besides DNA-binding activity, liposome leakage activity was crucial for successful gene transfer in vivo. Gene transfer with ppTG1 and ppTG20 was at least comparable to gene transfer with the optimized lipoplexes formed with pcTG90/DOPE 1:2 [+/-] 10.

Reporter gene activity was highest at day 1 after injection followed by a reduction over time. Re-administration at day 14 led to a renewal of reporter gene activity.

### Example 9 : DNA binding activity

ppTG1 derivatives as they are indicated in Materials and Methods, were tested for their capacity to bind plasmid DNA by gel retardation assays.

The results are summarized in the following Table 1

| | DNA binding activity | Liposome leakage assay | Transfection *in vitro* | Gene transfer *in vivo* |
|---|---|---|---|---|
| ppTG1 | + | + | + | + |
| ppTG20 | + | + | + | ++ |
| ppTG21 | + at pH5; - at pH8 | + | +/- | - |
| ppTG22 | + | + | +/- | +/- |
| ppTG23 | + | +/- | +/- | - |
| ppTG24 | + | +/- | - | - |
| ppTG25 | + | +/- | + | +/- |
| ppTG26 | + | +/- | + | +/- |
| ppTG27 | + | +/- | + | +/- |
| ppTG28 | + | +/- | + | - |
| ppTG29 | + | + | + | nd |
| ppTG30 | + | + | +/- | - |
| ppTG31 | + | +/- | +/- | nd |
| ppTG32 | + | - | - | - |
| ppTG33 | + | - | - | nd |
| ppTG1 non-purified | + | + | + | nd |
| PEG-ppTG1 non-purified | + | + | - | - |
| PEG-ppTG20 non-purified | + | + | - | - |
| ppTG20-D | + | + | + | ++ |

The results presented in Table 1 indicate that all peptides were capable to bind to plasmid DNA at pH8, except for ppTG21 (Lys⇒His). It has been, however, shown that ppTG21 can bind DNA at pH5. This result can be explained with the protonation status of the histidine residues (pK 6) and suggests that binding between plasmid DNA and ppTG1-derived peptides is mainly due to electrostatic interaction.

### Example 10: Liposome leakage activity

Liposome leakage activities were analyzed on liposomes consisting of POPC and cholesterol at a molar ratio of 3:2 (mol/mol). Cholesterol is an important ubiquitous component of natural membranes determining their fluidity. Tests with such liposomes are thus closer to in vivo conditions than tests with pure POPC liposomes. The peptides ppTG1, JTS-1-K13, KALA and JTS-1 were compared at pH5 and pH7.

The results are presented in Figure 9A.

Figure 9A clearly shows that KALA could not liberate calcein from cholesterol(chol)-containing liposomes. JTS-1-K13 was also impeded in calcein release at pH7, while low level release occurred at pH5. The pH-sensitive peptide JTS-1 showed high lysis activity at pH5, at pH7 this activity was reduced. ppTG1 could efficiently liberate calcein from POPC/chol liposomes at pH5 and pH7.

Complexes of ppTG1 with plasmid DNA pTG11236 were tested for liposome leakage activity on POPC/chol 3:2 liposomes.

The results are shown in Figure 9B.

Figure 9B clearly shows that at an excess of peptide over plasmid DNA (P/N 5 or 10), liposome leakage was comparable to free ppTG1. At charge ratios (P/N) of 1 or 0.8, conditions under which theoretically all peptides are engaged in binding to plasmid DNA, leakage activity was slightly reduced. This observation could be explained such that DNA-binding and membrane destructive activities require different structural properties.

The series of ppTG1 derivatives as they are listed in Materials and Methods was tested for liposome leakage activity on POPC/chol 3:2 liposomes at pH7. The results are shown in Figure 9C (ppTG20 and ppTG21), Figure 9D (ppTG22-ppTG24 and ppTG20-D), Figure 9 E (ppTG25-ppTG27,), Figure 9F (ppTG28 - ppTG33), and Figure 9 G (PEG-ppTG1 and PEG-ppTG20) are summarized in Table 1

The Figures 9C, 9D and 9G clearly show that the replacement of Lys in ppTG1 by Arg or His, ppTG20 in D-configuration or the addition of PEG2000 N-terminally to ppTG1 or ppTG20 did not reduce the membrane destructive activities of the resulting peptides. Figure 9F demonstrates that the addition of two basic amino acids (ppTG28 and ppTG29), or the replacement of Leu by Ile (ppTG31) slightly reduced liposome leakage activity. Replacement of Leu by Val significantly reduced liposome leakage activity (ppTG32 and ppTG33). Figure 9D indicates that additional Cys residues did not affect liposome leakage activity when added to the C-terminus, and slightly reduced membrane destruction, when added to the N-terminus (ppTG23 and ppTG24). Figure 9E clearly demonstrates that leakage activities with ppTG1 linked to wild type (wt), reversed or mutated NLS were significant, but below the maximal value obtained with ppTG1.

### Example 11: Transfection efficiency in vitro

The transfection efficiencies with plasmid / ppTG1 complexes were tested in the human tumor cells WiDr, MDA-MB-4355, SW480 and LoVo (ATCC) in comparison to lipofectin, PEI and pcTG90/DOPE (1:1) [+/-] 5. Luciferase activities at day 1 after transfection are shown in Figure 10A.

As Figure 10A clearly shows, ppTG1 / plasmid complexes can efficiently transfect human tumor cell lines, especially SW480 cells.

The series of ppTG1 derivatives listed in Material and Methods were tested for their gene transfer efficiencies in Hela cells. 6x10⁴ cells were transfected with 50 ng pTG11236 complexed with increasing amounts of peptide. Luciferase activity was analyzed day 1 after transfection. The results are shown in Figure 10B (ppTG20, ppTG21), Figure 10C(ppTG25, ppTG26, ppTG27), Figure 10D (ppTG28 to ppTG33), Figure 10E (PEG-ppTG1 and PEG-ppTG20), Figure 10F (ppTG22 to ppTG24) and Figure 10G (ppTG20-D). All results are also summarized in Table 1.

Figure 10B clearly shows that the replacement of Lys by Arg residues did not change the transfection efficiency, while replacing Lys by His resulted in significant reduction. Figure 10C demonstrates that the C-terminal addition of SV40 large T antigen-derived NLS peptide did not influence the efficiency of gene transfer. Figure 10D shows that the addition of two basic amino acid residues (ppTG28 and ppTG29) did not change transfection efficiencies. The replacement of Leu by Ile (ppTG30 and ppTG31), however, reduced the efficiency of gene transfer, and the replacement of Leu by Val reduced this activity even further. N-terminal, covalent addition of PEG-2000 reduced transfection efficiencies (Figure 10E), as well as Cys residues linked to the N and/or C-terminus of ppTG1 (Figure 10F). ppTG20-D, however, was as efficient as ppTG1 in gene transfer studies (Figure 10G).

### Example 11: Studies on the mechanism of gene transfer with ppTG1

Bafilomycin A is a specific inhibitor of the vacuolar proton pump. Treatment with Bafilomycin inhibits the acidification of late endosomes. HeLa cells were treated with Bafilomycin A (175 nM) 30 min before and throughout the transfection (1 h incubation with transfection complexes in the absence of serum). 6x10⁴ cells were transfected with 150 ng pTG11236 using PEI or ppTG1. The luciferase assay was performed 1 day after transfection.

The presence of Bafilomycin A did not influence the transfection efficiency with ppTG1, while transfection with PEI was 400-fold diminished (data not shown) . This indicates that, if ppTG1 / plasmid complexes are taken up by endosomes they are released via a pH-independent mechanism.

### Example 12: In vivo studies

The potential of gene transfer with mono-component peptide vectors was investigated *in vivo.* Fifty or sixty *µ*g of the luciferase expression plasmid pTG11236 were complexed with pcTG90 / DOPE (1:2) [+/-] 10 in 250 *µ*l 5% glucose (Meyer *et al.* 2000). The resulting lipoplex vector served as reference for gene transfer studies with pTG11236 complexed with ppTG1, ppTG20 and ppTG32 in 250 *µ*l 5% glucose. Five mice per group were intravenously injected, the animals were sacrificed at day 1 after injection. Lungs were tested for luciferase activity. The results are shown in FIGURE 11.

Figure 11A clearly demonstrates that gene transfer with ppTG1 complexes (at charge ratios [+/-] between 2 and 3) led to luciferase activities in the lung which were comparable to those obtained with the lipoplexes. Gene transfer with ppTG20 showed a general tendency to be more efficient and less toxic than ppTG1, while complexes with the peptide ppTG32 did not lead to detectable reporter gene expression. This implies that membrane-destructive activity is necessary for successful gene delivery with ppTG1-derived peptides.

Complexes with ppTG1 were compared to those formed with JTS-1-K13, KALA, K8-NLSm/JTS-1 and ppTG20. KALA and K8-NLSm/JTS-1 were inefficient (data not shown). Luciferase activities observed in the lung at day 1 after intravenous injection of 50 *µ*g pTG11236 complexed with ppTG1, JTS-1-K13 and ppTG20 are shown in Figure 11B. It appears that gene transfer with ppTG1 is better than with JTS-1-K13. Gene transfer with ppTG20 shows the reproducible tendency to give rise to higher gene expression and to be less toxic than ppTG1.

Gene expression obtained with ppTG1 / plasmid complexes dropped 3 days after injection to background levels (data not shown). Re-administration of ppTG1/plasmid complexes at day 14 after the first injection led to reappearance of reporter gene expression. At day 3 the system was still refractory (Figure 11C).

Figure 11D confirms that ppTG20 gives rise to more efficient gene transfer than ppTG1, while ppTG20-D seems to be even more efficient than ppTG20.

These data show for the first time mono-component peptide vectors which enable significant gene transfer *in vivo.*
Cited references :
Bartlett, G.R. 1959, *J. Biol. Chem.* 234, 466-469.
Gottschalk, S. et al., 1996, Gene Therapy 3 : 448-457.
Längle-Rouault et al. 1998. J. Virol. 72:6181-6185.
Mahato, R.I et al. 1999 *Current Opinions in Molecular Therapeutics 1,* 226-243.
Meyer, O. et al. (2000) Gene Therapy **7:**1606-1611.
Olson, F et al., 1979 *Biochim. Biophys. Acta* 557, 19-23.
Planck, C et al., 1994, *J. Biol. Chem.* 269, 12918-12924.
Wyman, T.B et al., 1997, Biochemistry 36 : 3008-3017

SEQUENCE LISTING

<110> Transgene S.A.
<120> Complex for transferring an anionic substance
of interest into a cell.
<130>
<140>
<141>
<160> 6
<170> PatentIn Ver. 2.1

<210> 1
<211> 20
<212> PRT
<213> Artificial Sequence
<220>
<223> Description of Artificial Sequence: mutPep
<400> 1
Gly Leu Phe Xaa Ala Leu Leu Xaa Leu Leu Xaa Ser Leu
1               5                   10
Trp Xaa Leu Leu Leu Xaa Ala
    15                  20

<210> 2
<211> 20
<212> PRT
<213> Artificial Sequence
<220>
<223> Description of Artificial Sequence: PPTG1
<400> 2
Gly Leu Phe Lys Ala Leu Leu Lys Leu Leu Lys Ser Leu
1               5                   10
Trp Lys Leu Leu Leu Lys Ala
    15                  20

<210> 3
<211> 20
<212> PRT
<213> Artificial Sequence
<220>
<223> Description of Artificial Sequence: JTS-1
<400> 3
Gly Leu Phe Glu Ala Leu Leu Glu Leu Leu Glu Ser Leu
  1               5                   10
Trp Glu Leu Leu Leu Glu Ala
    15                  20

<210> 4
<211> 40
<212> PRT
<213> Artificial Sequence
<220>
<223> Description of Artificial Sequence: JTS-1-K13
<400> 4
Gly Leu Phe Glu Ala Leu Leu Glu Leu Leu Glu Ser Leu
  1               5                   10
15
Trp Glu Leu Leu Leu Glu Ala Cys Cys Tyr Lys Ala Lys
     15                  20                      25

Lys Lys Lys Lys Lys Lys Lys Trp Lys Lys Lys Lys Gln
            30                  35
Ser
40

<210> 5
<211> 30
<212> PRT
<213> Artificial Sequence
<220>
<223> Description of Artificial Sequence: KALA

<400> 5
Trp Glu Ala Lys Leu Ala Lys Ala Leu Ala Lys Ala Leu
1               5                   10

Ala Lys His Leu Ala Lys Ala Leu Ala Lys Ala Leu Lys
15                  20                  25

Ala Cys Glu Ala
            30

<210> 6
<211> 20
<212> PRT
<213> Artificial Sequence
<220>
<223> Description of Artificial Sequence: ppTG20
<400> 2
Gly Leu Phe Arg Ala Leu Leu Arg Leu Leu Arg Ser Leu
1               5                   10
Trp Arg Leu Leu Leu Arg Ala
    15                  20

<210> 7
<211> 20
<212> PRT
<213> Artificial Sequence
<220>
<223> Description of Artificial Sequence:ppTG21
Gly-Leu-Phe-His-Ala-Leu-Leu-His-Leu-Leu-His-Ser-Leu-
Trp-His-Leu-Leu-Leu-His-Ala

<210> 8
<211> 21
<212> PRT
<213> Artificial Sequence
<220>
<223> Description of Artificial Sequence:ppTG22
Gly-Leu-Phe-Lys-Ala-Leu-Leu-Lys-Leu-Leu-Lys-Ser-Leu-
Trp-Lys-Leu-Leu-Leu-Lys-Ala-Cys

<210> 9
<211> 21
<212> PRT
<213> Artificial Sequence
<220>
<223> Description of Artificial Sequence:ppTG23
Cys-Gly-Leu-Phe-Lys-Ala-Leu-Leu-Lys-Leu-Leu-Lys-Ser-
Leu-Trp-Lys-Leu-Leu-Leu-Lys-Ala
<210> 10
<211> 22
<212> PRT
<213> Artificial Sequence
<220>
<223> Description of Artificial Sequence:ppTG24
Cys-Gly-Leu-Phe-Lys-Ala-Leu-Leu-Lys-Leu-Leu-Lys-Ser-
Leu-Trp-Lys-Leu-Leu-Leu-Lys-Ala-Cys

<210> 11
<211> 20
<212> PRT
<213> Artificial Sequence
<220>
<223> Description of Artificial Sequence:ppTG25
Gly-Leu-Phe-Lys-Ala-Leu-Leu-Lys-Leu-Leu-Lys-Ser-Leu-
Trp-Lys-Leu-Leu-Leu-Lys-Ala-Gly-Gly-Gly-Pro-Lys-Lys-
Lys-Arg-Lys-Val-Glu-Asp

<210> 12
<211> 20
<212> PRT
<213> Artificial Sequence
<220>
<223> Description of Artificial Sequence:ppTG26
Gly-Leu-Phe-Lys-Ala-Leu-Leu-Lys-Leu-Leu-Lys-Ser-Leu-
Trp-Lys-Leu-Leu-Leu-Lys-Ala-Gly-Gly-Gly-Pro-Lys-Thr-
Lys-Arg-Lys-Val-Glu-Asp
<210> 13
<211> 20
<212> PRT
<213> Artificial Sequence
<220>
<223> Description of Artificial Sequence:ppTG27
Gly-Leu-Phe-Lys-Ala-Leu-Leu-Lys-Leu-Leu-Lys-Ser-Leu-
Trp-Lys-Leu-Leu-Leu-Lys-Ala-Gly-Gly-Gly-Asp-Glu-Val-
Lys-Arg-Lys-Lys-Lys-Pro
<210> 14
<211> 20
<212> PRT
<213> Artificial Sequence
<220>
<223> Description of Artificial Sequence:ppTG28
Gly-Leu-Phe-Lys-Lys-Leu-Leu-Lys-Leu-Leu-Lys-Lys-Leu-
Trp-Lys-Leu-Leu-Leu-Lys-Ala
<210> 15
<211> 20
<212> PRT
<213> Artificial Sequence
<220>
<223> Description of Artificial Sequence:ppTG29
Gly-Leu-Phe-Arg-Arg-Leu-Leu-Arg-Leu-Leu-Arg-Arg-Leu-
Trp-Arg-Leu-Leu-Leu-Arg-Ala

<210> 16
<211> 20
<212> PRT
<213> Artificial Sequence
<220>
<223> Description of Artificial Sequence:ppTG30
Gly-Ile-Phe-Lys-Ala-Ile-Ile-Lys-Ile-Ile-Lys-Ser-Ile-
Trp-Lys-Ile-Ile-Ile-Lys-Ala
<210> 17
<211> 20
<212> PRT
<213> Artificial Sequence
<220>
<223> Description of Artificial Sequence:ppTG31
Gly-Ile-Phe-Arg-Ala-Ile-Ile-Arg-Ile-Ile-Arg-Ser-Ile-
Trp-Arg-Ile-Ile-Ile-Arg-Ala
<210> 18
<211> 20
<212> PRT
<213> Artificial Sequence
<220>
<223> Description of Artificial Sequence:ppTG32
Gly-Val-Phe-Lys-Ala-Val-Val-Lys-Val-Val-Lys-Ser-Val-
Trp-Lys-Val-Val-Val-Lys-Ala
<210> 19
<211> 20
<212> PRT
<213> Artificial Sequence
<220>
<223> Description of Artificial Sequence:ppTG33
Gly-Val-Phe-Arg-Ala-Val-Val-Arg-Val-Val-Arg-Ser-Val-
Trp-Arg-Val-Val-Val-Arg-Ala

<210> 20
<211> 20
<212> PRT
<213> Artificial Sequence
<220>
<223> Description of Artificial Sequence:ppTG20-D-
configuration
Gly-Leu-Phe-Arg-Ala-Leu-Leu-Arg-Leu-Leu-Arg-Ser-Leu-
Trp-Arg-Leu-Leu-Leu-Arg-Ala

## Claims

1. A cationic peptide which is capable of causing membrane disruption and which does not comprise acidic amino acid.

2. The peptide of claim 1 which does not comprise glutamic amino acid.

3. The peptide of claim 1 or 2 which has a molecular weight of less than 5 kD, preferably of less than 3 kD.

4. The peptide of any one of claims 1 to 3, which comprises the amino acid sequence SEQ ID NO:1, wherein each Xaa is selected independently of one another from the group consisting of lysine (Lys or K), histidine (His or H) and arginine (Arg or R) residues.

5. The peptide of claim 1, which comprises the amino acid sequence of SEQ ID NO:2 or SEQ ID NO:6, or selected in the group of SEQ ID NO: 7 to SEQ ID NO:20.

6. A complex for transferring an anionic substance of interest into a cell comprising:
(i) at least one peptide of any one of claims 1 to 5,
(ii) at least one anionic substance of interest.

7. The complex of claim 6, wherein said complex further comprises:
(iii) at least one ligand capable of cell-specific and/or nuclear targeting ; and/or
(iv) at least one further peptide which is capable of causing membrane disruption ; and/or
(v) at least one cationic compound selected from the group consisting of cationic lipids and cationic polymers ; and/or
(vi) at least one colipid.

8. The complex of claims 6 or 7, wherein said anionic substance of interest is a nucleic acid.

9. The complex of claim 8, wherein said nucleic acid comprises at least one therapeutically useful gene sequence and elements enabling its expression.

10. The complex of any one of claims 6 to 9, wherein the size of said complex is less than 500 nm.

11. The complex of claim 10, wherein said size is between 20 and 100 nm.

12. The complex of any one of claims 6 to 11, wherein the ratio within said complex between the number of positive charges and the number of negative charges is between 0.05 and 20.

13. The complex of claim 12, wherein said ratio is up to 1.

14. A composition comprising the complex of any one of claims 6 to 13.

15. Use of the complex of any one of claims 6 to 13 for the preparation of a pharmaceutical composition for curative, preventive or vaccine treatment of mammals.

16. Use of a peptide of any one of claims 1 to 5 for the preparation of a complex for transferring an anionic substance of interest into a cell.
